(19) Europäisches Patentamt / European Patent Office / Office européen des brevets

(11) **EP 4 714 449 A1**

(12) **EUROPEAN PATENT APPLICATION**
published in accordance with Art. 153(4) EPC

(43) Date of publication:
**25.03.2026 Bulletin 2026/13**

(21) Application number: **24810264.2**

(22) Date of filing: **14.05.2024**

(51) International Patent Classification (IPC):
***A61K 31/506*** *(2006.01)* ***C07D 487/08*** *(2006.01)*
***A61P 35/00*** *(2006.01)*

(52) Cooperative Patent Classification (CPC):
**A61K 31/506; A61P 35/00; C07D 487/08**

(86) International application number:
**PCT/CN2024/093044**

(87) International publication number:
**WO 2024/240017 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.05.2023 CN 202310567443**

(71) Applicant: **Sichuan Kelun-Biotech Biopharmaceutical Co., Ltd.**
**Chengdu, Sichuan 611138 (CN)**

(72) Inventors:
- **QING, Yan**
  **Chengdu, Sichuan 611138 (CN)**
- **YIN, Li**
  **Chengdu, Sichuan 611138 (CN)**
- **XIANG, Xingping**
  **Chengdu, Sichuan 611138 (CN)**
- **YAN, Shan**
  **Chengdu, Sichuan 611138 (CN)**
- **HE, Qiang**
  **Chengdu, Sichuan 611138 (CN)**
- **LIU, Guangpan**
  **Chengdu, Sichuan 611138 (CN)**
- **JIN, Xiaoping**
  **Chengdu, Sichuan 611138 (CN)**
- **GE, Junyou**
  **Chengdu, Sichuan 611138 (CN)**

(74) Representative: **Hoffmann Eitle**
**Patent- und Rechtsanwälte PartmbB**
**Arabellastraße 30**
**81925 München (DE)**

(54) **USE OF HETEROCYCLIC COMPOUND FOR TREATING DISEASES RELATED TO RET GENETIC CHANGE AND METHOD THEREFOR**

(57)

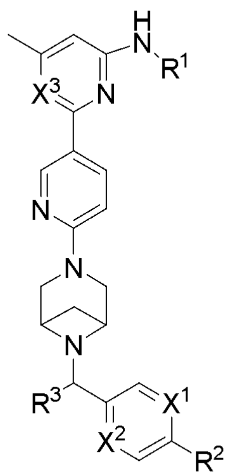

**I**

The use of a heterocyclic compound of formula I in the preparation of a drug for treating RET-changed tumors and a treatment method therefor. The compound of formula I has good safety and tolerability in terms of treating non-small cell lung cancer (NSCLC), medullary carcinoma of thyroida (MTC), papillary carcinoma of thyroid (PTC), pancreatic cancer, ovarian cancer, inhibitor drug-resistant solid tumor and/or neuroendocrine colorectal cancer (CRC).

## Description

The present application is based on CN application number 202310567443.8, filed on May 19, 2023, and claims priority thereof. The disclosure of this CN application is hereby incorporated herein by reference in its entirety.

## FIELD OF THE INVENTION

The present disclosure relates to use of heterocyclic compounds in the manufacture of a medicament for treating tumors associated with RET alterations, as well as the corresponding treatment methods.

## BACKGROUND OF THE INVENTION

Protein kinases are a class of enzymes catalysing protein phosphorylation reactions. By mediating the process of cell signal transduction, protein phosphorylation regulates the physiological activities of cells, such as cell survival, proliferation, differentiation, apoptosis, and metabolism. The dysfunction of the protein kinases is closely associated with many diseases, including tumours, autoimmune diseases, inflammatory reactions, central nervous system diseases, cardiovascular diseases, diabetes, and the like.

As a protooncogene, RET encodes an RET protein which is a transmembrane receptor type tyrosine protein kinase consisting of a cysteine-rich cadherin-like extracellular domain (for binding to ligands), a transmembrane domain, and an intracellular domain with tyrosine kinase activity. The RET gene encodes a cell surface signaling receptor that regulates cell differentiation, growth, and migration, and is implicated in the pathogenesis of approximately 2% of human cancers.

Current treatments for RET+ tumors largely follow recommended guidelines, such as first-line treatment with chemoimmunotherapy, individual chemotherapy or immunotherapy, or marketed RET inhibitors. Second-line and beyond treatments employ single-agent chemotherapy, single-agent immunotherapy, or marketed RET inhibitors. Standard chemotherapy and/or immunotherapy exhibit limited efficacy, and prolonged use of marketed RET inhibitors leads to RET resistance mutations, resulting in reduced efficacy, recurrence, and poor prognosis. Due to resistance limitations, the therapeutic effects of marketed RET inhibitors are constrained, and safety concerns such as hypertension and hematologic toxicity, *etc.* persist during the treatment. Other multi-target drugs used to treat RET gene fusion mutations lack strong specificity for RET gene fusion mutations, which results in relatively limited effectiveness.

Therefore, there is a need to explore treatment methods and uses of RET inhibitors that improve safety and efficacy and can overcome resistance mutations.

## SUMMARY OF THE INVENTION

In one aspect, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET altered tumors:

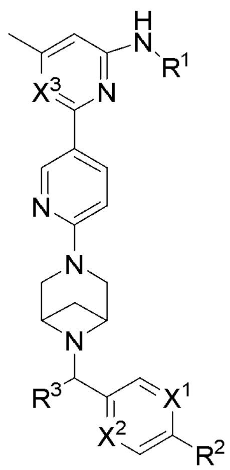

Formula **I**

wherein:

$R^1$ is selected from the group consisting of 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and

$X^1$, $X^2$ and $X^3$ are each independently selected from the group consisting of CH and N.

In certain embodiments, the present disclosure provides use of Compound 1, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET altered tumors, wherein Compound 1 has the following structure:

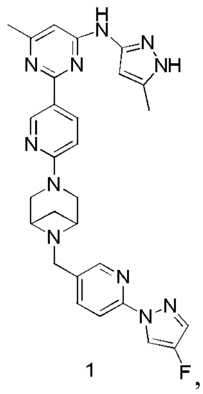

1 ,

for example, the pharmaceutically acceptable salt of Compound 1 is the fumarate salt of Compound 1.

In certain embodiments, the RET alteration is a RET fusion or RET mutation.

In certain embodiments, the RET fusion is selected from KIF5B-RET fusion, CCDC6-RET fusion, NCOA4-RET fusion, RELCH-RET fusion, and ERC1-RET fusion. In certain embodiments, the RET mutation is selected from G810S, G810R, G810C, V804M, V804L, and V804E.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET+ solid tumors.

In certain embodiments, the RET+ solid tumor is a RET fusion-positive or RET mutation-positive solid tumor.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E.

In certain embodiments, the RET fusion-positive or RET mutation-positive solid tumor is selected from non-small cell lung cancer (NSCLC), medullary thyroid carcinoma (MTC), papillary thyroid carcinoma (PTC), pancreatic cancer, ovarian cancer, inhibitor resistant solid tumors, and neuroendocrine colorectal cancer (neuroendocrine CRC).

In certain embodiments, the RET fusion-positive or RET mutation-positive solid tumor is selected from non-small cell lung cancer (NSCLC), medullary thyroid carcinoma (MTC), papillary thyroid carcinoma (PTC), pancreatic cancer, ovarian cancer, and inhibitor resistant solid tumors.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of non-small cell lung cancer (NSCLC), medullary thyroid carcinoma (MTC), papillary thyroid carcinoma (PTC), pancreatic cancer, ovarian cancer, inhibitor resistant solid tumors, and/or neuroendocrine colorectal cancer (neuroendocrine CRC).

In certain embodiments, the present disclosure provides use of a compound of Formula I, a stereoisomer,

tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of non-small cell lung cancer (NSCLC), medullary thyroid carcinoma (MTC), papillary thyroid carcinoma (PTC), pancreatic cancer, ovarian cancer, and/or inhibitor resistant solid tumors. In certain embodiments, the medicament is one for treating RET+ non-small cell lung cancer, RET+ medullary thyroid carcinoma, RET+ papillary thyroid carcinoma, RET+ pancreatic cancer, RET+ ovarian cancer, and/or inhibitor resistant solid tumors.

In certain embodiments, the medicament is one for treating RET fusion-positive non-small cell lung cancer, RET fusion-positive medullary thyroid carcinoma, RET fusion-positive papillary thyroid carcinoma, RET fusion-positive pancreatic cancer, RET fusion-positive ovarian cancer, RET fusion-positive neuroendocrine colorectal cancer (neuroendocrine CRC), and/or RET fusion-positive inhibitor resistant solid tumors.

In certain embodiments, the medicament is one for treating RET fusion-positive non-small cell lung cancer, RET fusion-positive medullary thyroid carcinoma, RET fusion-positive papillary thyroid carcinoma, RET fusion-positive pancreatic cancer, RET fusion-positive ovarian cancer, and/or RET fusion-positive inhibitor resistant solid tumors.

In certain embodiments, the RET fusion is selected from KIF5B-RET fusion, CCDC6-RET fusion, NCOA4-RET fusion, RELCH-RET fusion, and ERC1-RET fusion.

In certain embodiments, the medicament is one for treating RET mutation-positive non-small cell lung cancer, RET mutation-positive medullary thyroid carcinoma, RET mutation-positive papillary thyroid carcinoma, RET mutation-positive pancreatic cancer, RET mutation-positive ovarian cancer, RET mutation-positive neuroendocrine colorectal cancer (neuroendocrine CRC), and/or RET mutation-positive inhibitor resistant solid tumors.

In certain embodiments, the medicament is one for treating RET mutation-positive non-small cell lung cancer, RET mutation-positive medullary thyroid carcinoma, RET mutation-positive papillary thyroid carcinoma, RET mutation-positive pancreatic cancer, RET mutation-positive ovarian cancer, and/or RET mutation-positive inhibitor resistant solid tumors.

In certain embodiments, the RET mutation is selected from G810S, G810R, G810C, V804M, V804L, and V804E mutations.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET+ non-small cell lung cancer (NSCLC) as a first-line or second-line or beyond therapy.

In certain embodiments, the RET+ non-small cell lung cancer (NSCLC) is RET fusion-positive or RET mutation-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In another aspect, the present disclosure provides a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET-altered tumors.

In certain embodiments, the RET-altered tumor is a RET+ solid tumor.

In certain embodiments, the RET+ solid tumor is a RET fusion-positive or RET mutation-positive solid tumor.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In certain embodiments, the RET+ solid tumor is selected from non-small cell lung cancer (NSCLC), medullary thyroid carcinoma (MTC), papillary thyroid carcinoma (PTC), pancreatic cancer, ovarian cancer, neuroendocrine colorectal cancer (neuroendocrine CRC), and inhibitor resistant solid tumors.

In certain embodiments, the RET+ solid tumor is selected from non-small cell lung cancer (NSCLC), medullary thyroid carcinoma (MTC), papillary thyroid carcinoma (PTC), pancreatic cancer, ovarian cancer, and inhibitor resistant solid tumors.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the present disclosure provides a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET+ non-small cell lung cancer (NSCLC) as a first-line or second-line therapy.

In certain embodiments, the RET+ non-small cell lung cancer (NSCLC) is RET fusion-positive or RET mutation-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In another aspect, the present disclosure provides a method for treating RET-altered tumors, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof.

In certain embodiments, the daily dose of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is 10-120 mg, such as 10 mg, 20 mg, 40 mg, 60 mg, 90 mg, 120 mg, 20-120 mg, 30-120 mg, 40-120 mg, 50-120 mg, 60-120 mg, 70-120 mg, 80-120 mg, 90-120 mg, 100-120 mg or 110-120 mg.

In certain embodiments, the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is administered at a dosing schedule of once every 1-7 days, for example, once daily, once every 2 days, once every 3 days, once every 4 days, once every 5 days, once every 6 days, or once every 7 days.

In certain embodiments, the dosing schedule is administration once daily at a daily dose of 40-120 mg.

In certain embodiments, the RET-altered tumor is a RET+ solid tumor, such as non-small cell lung cancer (NSCLC), medullary thyroid carcinoma (MTC), papillary thyroid carcinoma (PTC), pancreatic cancer, ovarian cancer, neuroendocrine colorectal cancer (neuroendocrine CRC), inhibitor resistant solid tumor.

In certain embodiments, the RET-altered tumor is a RET+ solid tumor, such as non-small cell lung cancer (NSCLC), medullary thyroid carcinoma (MTC), papillary thyroid carcinoma (PTC), pancreatic cancer, ovarian cancer, inhibitor resistant solid tumor.

In certain embodiments, the RET-altered tumor is a RET fusion-positive solid tumor, such as non-small cell lung cancer (NSCLC), medullary thyroid carcinoma (MTC), papillary thyroid carcinoma (PTC), pancreatic cancer, ovarian cancer, inhibitor resistant solid tumor, neuroendocrine colorectal cancer (neuroendocrine CRC).

In certain embodiments, the RET fusion-positive solid tumor is RET fusion-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the objective response rate (ORR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 60%.

In certain embodiments, the objective response rate (ORR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 63%, such as 64%.

In certain embodiments, the objective response rate (ORR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 70%, 81%.

In certain embodiments, the disease control rate (DCR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 90%, such as 94%, 96%.

In another aspect, the present disclosure provides a method for treating RET+ non-small cell lung cancer

(NSCLC) as a first-line or second-line or beyond therapy, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof.

In certain embodiments, the RET+ non-small cell lung cancer (NSCLC) is RET fusion-positive or RET mutation-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In certain embodiments, the subject is treatment-naive or is systemically pretreated, such as pretreated with anti-PD1/PD-L1 therapy, other RET inhibitor therapy (e.g., selpercatinib, pralsetinib, SY5007, YP01001 or HA121-28), MKI inhibitor therapy (e.g., anlotinib, cabozantinib, apatinib or CX1003), or chemotherapy.

In certain embodiments, the subject is treatment-naive or is systemically pretreated, such as pretreated with anti-PD1/PD-L1 therapy, other RET inhibitor therapy, MKI inhibitor therapy, or chemotherapy.

In certain embodiments, the subject is treatment-naive or is systemically pretreated, such as pretreated with anti-PD1/PD-L1 therapy, other RET selective inhibitor therapy, MKI inhibitor therapy, or chemotherapy.

In certain embodiments, the subject is treatment-naive or is systemically pretreated, such as pretreated with anti-PD1/PD-L1 therapy, selpercatinib, pralsetinib, SY5007, anlotinib, cabozantinib, apatinib, CX1003, or chemotherapy.

In certain embodiments, the subject is systemically pretreated, such as pretreated with anti-PD1/PD-L1 therapy, other RET inhibitor therapy (e.g., selpercatinib, pralsetinib, SY5007, YP01001, or HA121-28), MKI inhibitor therapy (e.g., anlotinib, cabozantinib or apatinib, CX1003), or chemotherapy.

In certain embodiments, the subject is systemically pretreated, such as pretreated with anti-PD1/PD-L1 therapy, other RET selective inhibitor therapy (e.g., selpercatinib, pralsetinib, or SY5007), MKI inhibitor therapy (e.g., anlotinib, cabozantinib or apatinib, CX1003), or chemotherapy.

In certain embodiments, the subject is systemically pretreated, such as pretreated with anti-PD1/PD-L1 therapy, other RET selective inhibitor therapy, MKI inhibitor therapy, or chemotherapy.

In certain embodiments, the subject is systemically pretreated, such as pretreated with anti-PD1/PD-L1 therapy, selpercatinib, pralsetinib, SY5007, YP01001, HA121-28, anlotinib, cabozantinib or apatinib, CX1003, or chemotherapy.

In preferred embodiments, the subject has a median prior treatment number selected from 1-15, such as 1-12, such as 1-10, such as 1-9, such as 1-7, such as 1, 2, 3, 4, 5, 6, 7, 8, 9, 10, 11, 12, 13, 14, 15.

In preferred embodiments, the subject has a median prior treatment number selected from 1-9, such as 1, 2, 3, 4, 5, 6, 7, 8, 9.

In certain embodiments, the subject has been previously treated with anti-PD1 or PD-L1 systemic therapy.

In preferred embodiments, the disease control rate (DCR) of the subject is at least 91%.

In preferred embodiments, the disease control rate (DCR) of the subject is at least 94%, such as 97%.

In preferred embodiments, the subject has an objective response rate of at least 60%, and a disease control rate of at least 94%.

In preferred embodiments, the subject has an objective response rate of at least 63%, and a disease control rate of at least 91%.

In preferred embodiments, the subject has an objective response rate of at least 70%, and a disease control rate of at least 97%.

In certain embodiments, the subject has non-small cell lung cancer (NSCLC) with brain metastasis, bone metastasis, or liver metastasis.

In certain embodiments, the subject has non-small cell lung cancer (NSCLC) with brain metastasis, and further, the subject has not been previously treated with radiation therapy.

In preferred embodiments, the subject's measurable brain lesions exhibit at least a 17% reduction.

In preferred embodiments, the subject's measurable brain lesions exhibit at least a 47% reduction, such as 80%, 100%.

In preferred embodiments, the subject's measurable brain lesions exhibit at least a 69% reduction.

In preferred embodiments, the subject's measurable brain lesions exhibit at least a 47% reduction, such as 80%, 100%, and the overall central nervous system disease control rate (CNS DCR) is 100%.

In preferred embodiments, the subject's measurable brain lesions exhibit at least a 17% reduction, and the overall central nervous system disease control rate (CNS DCR) is 100%.

In certain embodiments, the subject has been previously treated with other RET inhibitor therapy (e.g., selpercatinib, pralsetinib, SY5007, YP01001, and/or HA121-28).

In certain embodiments, the subject has been previously treated with other RET inhibitor therapy (e.g., selpercatinib, pralsetinib, SY5007, YP01001, or HA121-28).

In certain embodiments, the subject has been previously treated with other RET selective inhibitor therapy (e.g., selpercatinib, pralsetinib, or SY5007).
In certain embodiments, the subject is a patient who has been previously treated with other RET selective inhibitors and has developed resistance.
In certain embodiments, the resistance is to selpercatinib, pralsetinib, and/or SY5007.
In certain embodiments, the subject who has been previously treated with other RET selective inhibitors has an objective response rate (ORR) of at least 43%, and further, the subject's disease control rate (DCR) is at least 86%.
In certain embodiments, the subject who has been previously treated with other RET selective inhibitors has an objective response rate (ORR) of at least 38%, and further, the subject's disease control rate (DCR) is at least 88%.
In certain embodiments, the subject who has been previously treated with other RET selective inhibitors is a patient positive for KIF5B-RET fusion, CCDC6-RET fusion, NCOA4-RET fusion, RELCH-RET fusion, and/or ERC1-RET fusion.
In certain embodiments, the subject who has been previously treated with other RET selective inhibitors is a patient positive for G810S, G810R, G810C, V804M, V804L, and/or V804E mutations.
In certain embodiments, the subject has been previously treated with MKI inhibitor therapy (e.g., anlotinib, cabozantinib or apatinib, CX1003).
In certain embodiments, the subject has been previously treated with chemotherapy.
In certain embodiments, the subject is not systemically pretreated, such as pretreated with anti-PD1/PD-L1 therapy, other RET selective inhibitor therapy (e.g., selpercatinib or pralsetinib), or chemotherapy.
In some embodiments, the objective response rate (ORR) of the subject who is not systemically pretreated is at least 60%.
In some embodiments, the objective response rate (ORR) of the subject who is not systemically pretreated is at least 74%, such as 76%, 77%.
In some embodiments, the objective response rate (ORR) of the subject who is not systemically pretreated is at least 81%.
In some embodiments, the disease control rate (DCR) of the subject who is not systemically pretreated is at least 92%.
In some embodiments, the disease control rate (DCR) of the subject who is not systemically pretreated is at least 96%.
In certain embodiments, the subject who is not systemically pretreated is selected from a subject who has not been previously treated with other RET selective inhibitors.
In certain embodiments, the subject who has not been previously treated with other RET selective inhibitors is a patient positive for KIF5B-RET fusion, CCDC6-RET fusion, NCOA4-RET fusion, RELCH-RET fusion, and/or ERC1-RET fusion.
In certain embodiments, the subject who has not been previously treated with other RET selective inhibitors is a patient positive for G810S, G810R, G810C, V804M, V804L, and/or V804E mutations.
In another aspect, the present disclosure provides a method for treating a patient with a medicament, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, according to one or both of the following dosing regimens:

(1) a daily dose of 10-120 mg, such as 20-120 mg, 30-120 mg, 40-120 mg, 50-120 mg, 60-120 mg, 70-120 mg, 80-120 mg, 90-120 mg, 100-120 mg, or 110-120 mg; and/or
(2) administration once every 1-7 days, such as once daily, once every 2 days, once every 3 days, once every 4 days, once every 5 days, once every 6 days, or once every 7 days;

after treatment, evaluating the subject's indicators to ensure no occurrence of Grade 3 or higher treatment-related adverse events (TRAE), or that majority of reversible treatment-related adverse events (TRAE) are less than Grade 3.
In certain embodiments, the "majority" is defined as greater than 70%.
In certain embodiments, the "majority" is defined as greater than 74.4%.
In certain embodiments, the "majority" is defined as 76.1%.
In certain embodiments, the treatment-related adverse events less than Grade 3 are selected from elevated aspartate aminotransferase (AST), elevated alanine aminotransferase (ALT), increased creatinine, increased bilirubin, anemia, constipation, and headache.
In certain embodiments, the treatment-related adverse events less than Grade 3 are selected from elevated aspartate aminotransferase (AST), elevated alanine aminotransferase (ALT), increased creatinine, increased bilirubin, constipation, and headache.

In certain embodiments, the treatment-related adverse events of Grade 3 or higher are selected from increased alkaline phosphatase (ALP), increased gamma-glutamyl transferase (GGT), and intestinal obstruction.
In preferred embodiments, hypertension, QT interval prolongation, thrombocytopenia, and lymphocytopenia are rare (e.g., <5%) or of low grade.
In preferred embodiments, hypertension, QT interval prolongation, and lymphocytopenia are rare (e.g., <5%) or of low grade.
The compound of the present disclosure demonstrates clear efficacy against RET-altered solid tumors and has good inhibitory effects on RET-resistant mutations, making them suitable for treating subjects who are resistant to existing selective RET inhibitors and have central nervous system brain metastasis (CNS Mets). Compared to existing selective RET inhibitors, the compound exhibits better tolerability and safety, with rare occurrences of hypertension, QT interval prolongation, thrombocytopenia, and lymphocytopenia.

## DETAILED DESCRIPTION OF THE INVENTION

### Definitions

Unless otherwise defined in the context, all technical terms and scientific terms used herein are intended to have the same meaning as commonly understood by those skilled in the art. The reference to a technology used herein is intended to refer to a technology generally understood in the art, including those technological alterations or equivalent technological replacements that are obvious to those skilled in the art. While it is believed that the following terms are well understood by those skilled in the art, the following definitions are still set forth to better explain the present disclosure.
The term "including," "comprising," "having," "containing," or "relating to" and additional variations thereof herein are inclusive or open-ended, and do not exclude additional unlisted elements or method steps, although additional unlisted elements or method steps do not necessarily exist (i.e., these terms also encompass the terms "substantially consisting of' and "consisting of").
As used herein, the term "alkyl" is defined as a linear or branched saturated aliphatic hydrocarbon. In some embodiments, aryl has from 1 to 12, e.g., from 1 to 6, carbon atoms. For example, as used herein, the terms "$C_{1-6}$ alkyl" and "$C_{1-4}$ alkyl" refer to a linear or branched group having from 1 to 6 carbon atoms and a linear or branched radical having from 1 to 4 carbon atoms respectively (e.g., methyl, ethyl, n-propyl, isopropyl, n-butyl, isobutyl, sec-butyl, tert-butyl, n-pentyl, isopentyl, neopentyl, or n-hexyl), which are optionally substituted with one or more (e.g., from 1 to 3) suitable substituents, e.g., halogen (in this case, the radical is termed "haloalkyl") (e.g., $CH_2F$, $CHF_2$, $CF_3$, $CCl_3$, $C_2F_5$, $C_2Cl_5$, $CH_2CF_3$, $CH_2Cl$, or - $CH_2CH_2CF_3$). The term "$C_{1-4}$ alkyl" refers to a linear or branched aliphatic hydrocarbon chain having from 1 to 4 carbon atoms (i.e., methyl, ethyl, n-propyl, isopropyl, n-butyl, isobutyl, sec-butyl, or tert-butyl).
As used herein, the term "heteroalkyl" refers to an optionally substituted alkyl radical that has one or more backbone chain atoms selected from atoms other than carbon, such as oxygen, nitrogen, sulphur, phosphorus, or combinations thereof. A numerical range (e.g., $C_{1-6}$ heteroalkyl) that may be given refers to the number of carbons in a chain, including from 1 to 6 carbon atoms in this example. For example, - $CH_2OCH_2CH_3$ group is termed $C_3$ heteroalkyl. Its attachment to the rest of the molecule may be through a heteroatom or carbon atom in the heteroalkyl chain.
As used herein, the term "haloalkyl" refers to an alkyl radical substituted with one or more (e.g., from 1 to 3) same or different halogen atoms, and the term "$C_{1-6}$ haloalkyl" and "$C_{1-4}$ haloalkyl" refer to a haloalkyl radical having from 1 to 6 carbon atoms and a haloalkyl radical having from 1 to 4 carbon atoms respectively, such as $-CF_3$, $-C_2F_5$, $-CHF_2$, $-CH_2F$, $-CH_2CF_3$, $-CH_2Cl$, or $-CH_2CH_2CF_3$ and the like.
As used herein, the term "hydroxyalkyl" refers to a group formed by substituting hydrogen atom(s) in an alkyl radical with one or more hydroxy, e.g., $C_{1-4}$ hydroxyalkyl or $C_{1-3}$ hydroxyalkyl, and its examples include, but are not limited to, hydroxymethyl, hydroxyethyl, hydroxypropyl, hydroxybutyl, $-CH(OH)CH_3$, $-C(CH_3)_2OH$, and the like.
As used herein, the term "alkoxy" refers to a group formed by inserting an oxygen atom into any reasonable position of an alkyl radical (as defined above), and is, for example, $C_{1-6}$ alkoxy, $C_{1-4}$ alkoxy, or $C_{1-3}$ alkoxy. Representative examples of $C_{1-6}$ alkoxy include, but are not limited to, methoxy, ethoxy, propoxy, isopropoxy, n-propoxy, isopropoxy, n-butoxy, isobutoxy, tert-butoxy, pentyloxy, hexyloxy, $-CH_2-OCH_3$, and the like, and the alkoxy is optionally substituted with one or more (e.g., from 1 to 3) same or different substituents.
As used herein, the term "condensed ring" or "fused ring" refers to a ring system formed by two or more ring structures sharing two adjacent atoms with each other.
As used herein, the term "spiro ring" refers to a ring system formed by two or more ring structures sharing one ring atom with each other.
As used herein, the term "bridged ring" refers to a ring system formed by two or more ring structures sharing two atoms (that are not directly connected) with each other.
As used herein, the term "cycloalkyl" refers to a saturated or unsaturated non-aromatic monocyclic or multicyclic (such as bicyclic) hydrocarbon ring radical, including but not limited to monocycloalkyl (such as cyclopropyl, cyclobutyl,

cyclopentyl, cyclohexyl, cycloheptyl, cyclooctyl, and cyclononyl) and bicycloalkyl, including a spiro ring, condensed (fused) ring, or bridged ring system (i.e., spirocycloalkyl, condensed (fused) cycloalkyl, and bridged cycloalkyl, such as bicyclo[1.1.1]pentyl, and bicyclo[2.2.1]heptyl). In the present disclosure, a cycloalkyl radical is optionally substituted with one or more (e.g., from 1 to 3) same or different substituents. A carbon atom on a cycloalkyl radical is optionally substituted with an oxo group (i.e., forming C=O). The term "$C_{3-6}$ cycloalkyl" refers to a cycloalkyl radical having from 3 to 6 ring-forming carbon atoms, which may be a monocycloalkyl radical, such as cyclopropyl, cyclobutyl, cyclopentyl, cyclohexyl, cycloheptyl, or cyclooctyl, and may also be a bicycloalkyl radical, such as $C_{5-8}$ spirocycloalkyl, $C_{5-8}$ bridged cycloalkyl, $C_{5-8}$ fused cycloalkyl, $C_{5-6}$ spirocycloalkyl, $C_{5-6}$ bridged cycloalkyl, or $C_{5-6}$ fused cycloalkyl.

As used herein, the term "cycloalkoxy" means -O-cycloalkyl, where the cycloalkyl is as defined above. Representative examples of cycloalkoxy radicals include, but are not limited to, cyclopropoxy, cyclobutoxy, cyclopentoxy, cyclohexoxy, and the like.

As used herein, the term "heterocyclyl" or "heterocyclic ring" refers to a monocyclic or multicyclic (for example, condensed, spiro, or bridged cyclic) radical having 2 or more than 2 (e.g., 3, 4, 5, 6, 7, 8, 9, 10, 11, 12, 13, or 14) carbon atoms, and one or more (for example, 1, 2, 3, or 4) heteroatoms, wherein the heteroatoms include, but are not limited to, an oxygen atom, a nitrogen atom, and a sulphur atom, and the carbon atoms and heteroatoms on the heterocyclyl are optionally substituted with an oxo group (for example, forming C=O, S(=O), or $S(=O)_2$).

As used herein, the term "4-10-membered heterocyclyl" means a heterocyclyl radical containing from 4 to 10 ring atoms, including but not limited to, 4-9-membered heterocyclyl, 4-8-membered heterocyclyl, 4-7-membered heterocyclyl, 5-6-membered heterocyclyl, 3-8-membered heterocyclyl, 3-7-membered heterocyclyl, 4-7-membered nitrogen-containing heterocyclyl, 4-7-membered oxygen-containing heterocyclyl, 4-7-membered sulphur-containing heterocyclyl, 5-6-membered nitrogen-containing heterocyclyl, 5-6-membered oxygen-containing heterocyclyl, 5-6-membered sulphur-containing heterocyclyl, and the like. The "nitrogen-containing heterocyclyl," "oxygen-containing heterocyclyl," and "sulphur-containing heterocyclyl" each optionally further contain one or more additional heteroatoms selected from oxygen, nitrogen, and sulphur. Examples of 4-10-membered heterocyclyl include, but are not limited to, oxiranyl, aziridinyl, azacyclobutyl, oxacyclobutyl, tetrahydrofuranyl, pyrrolidinyl, pyrrolidonyl (e.g.,

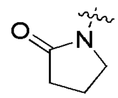

), imidazolidinyl, pyrazolidinyl, tetrahydropyranyl, piperidinyl, morpholinyl, dithianyl, thiomorpholinyl, piperazinyl, and trithianyl.

As used herein, the term "heteroaryl" or "heteroaromatic ring" refers to a monocyclic or multicyclic aromatic group comprising one or more same or different heteroatoms, including a monocyclic heteroaryl radical and a bicyclic or multicyclic ring system comprising at least one heteroaromatic ring (an aromatic ring system comprising at least one heteroatom), which may have 5, 6, 7, 8, 9, 10, 11, 12, 13, or 14 ring atoms, for example, 5, 6, 7, 8, 9, or 10 ring atoms. The heteroatom may be oxygen, nitrogen, or sulphur. A carbon atom and a heteroatom on the heteroaryl are optionally substituted with an oxo group (for example, forming C=O, S(=O), or $S(=O)_2$).

As used herein, the term "5-10-membered heteroaryl" or "5-10-membered heteroaromatic ring" means a heteroaryl radical (heteroaromatic ring) comprising from 5 to 10 (e.g., from 5 to 6) ring atoms, including 5-10-membered nitrogen-containing heteroaryl, 5-10-member oxygen-containing heteroaryl, 5-10-membered sulphur-containing heteroaryl, 5-6-membered nitrogen-containing heteroaryl, 5-6-membered oxygen-containing heteroaryl, 5-6-membered sulphur-containing heteroaryl, and the like. The "nitrogen-containing heteroaryl," "oxygen-containing heteroaryl," and "sulphur-containing heteroaryl" each optionally comprise one or more additional heteroatoms selected from oxygen, nitrogen, and sulphur. Examples thereof include, but are not limited to, thienyl, furanyl, pyrrolyl, oxazolyl, thiazolyl, imidazolyl, pyrazolyl, isoxazolyl, isothiazolyl, triazolyl, tetrazolyl, oxadiazolyl, thiadiazolyl, etc., or pyridyl, pyridazinyl, pyrimidinyl, pyrazinyl, triazinyl, etc., and a 5-10-membered condensed ring group comprising these radicals.

As used herein, the term "heteroaryl" encompasses a condensed ring structure, and the point of attachment of the condensed ring structure to additional radicals may be on any ring of the condensed ring structure. Therefore, heteroaryl radicals of the present disclosure further include, but are not limited to, (mono)heteroaryl-(mono)heteroaryl, (mono)heteroaryl-(monocyclo)aryl, (mono)heteroaryl-(mono)heterocyclyl, and (mono)heteroaryl-(mono)cycloalkyl, such as 5-6-membered (mono)heteroaryl-5-6-membered (mono)heteroaryl, 5-6 membered (mono)heteroaryl-phenyl, 5-6-membered (mono)heteroaryl-5-6-membered (mono)heterocyclyl, or 5-6-membered (mono)heteroaryl-$C_{4-6}$(mono)cycloalkyl (e.g., 5-6-membered heteroaryl-cyclobutyl, 5-6-membered heteroaryl-cyclopentyl, or 5-6-membered heteroaryl-cyclohexyl). Examples of heteroaryl include, but are not limited to, indolyl, isoindolyl, indazolyl, benzimidazolyl, quinolinyl, isoquinolinyl,

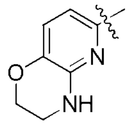, 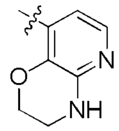, 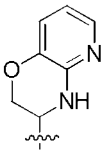, 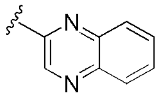,

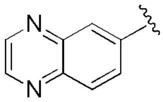, 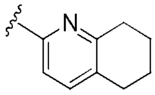, 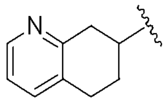, 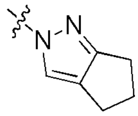,

and the like.

As used herein, the term "halo" or "halogen" radical is defined to encompass F, Cl, Br, or I.

The term "substitution" means that one or more (e.g., one, two, three, or four) hydrogens on a specified atom are replaced by a selection from the indicated radicals, provided that the normal valence of the specified atom in the present case is not exceeded and the substitution leads to a stable compound. A combination of substituents and/or variables is permissible only when such a combination leads to a stable compound.

If a substituted radical is described as "optionally...substituted," the substituted radical may be (1) unsubstituted, or (2) substituted. If a carbon of a substituted radical is described as being optionally substituted with one or more substituents in a list of substituents, one or more hydrogens (to the extent of any present hydrogens) on the carbon may be independently and/or together replaced with independently selected optional substituents. If a nitrogen of a substituted radical is described as being optionally substituted with one or more substituents in a list of substituents, one or more hydrogens (to the extent of any present hydrogens) on the nitrogen may each be replaced with independently selected optional substituents.

If a substituent is described as being "independently selected from" a group, each substituent is selected independently of the others. Therefore, each substituent may be the same as or different from another (other) substituent(s).

As used herein, the term "one or more" means 1 or more than 1, such as 2, 3, 4, 5, or 10, under reasonable conditions.

Unless otherwise specified, as used herein, the points of attachment of a substituent may be from any suitable positions at the substituent.

When a bond of a substituent is shown as a bond connecting two atoms through a ring, such a substituent may bond to any ring-forming atom in the substitutable ring.

The present disclosure further includes all pharmaceutically acceptable isotopically-labelled compounds, which are the same as the compounds of the present disclosure, except that one or more atoms are replaced with atoms which have the same atomic number, but an atomic mass or mass number different from the atomic mass or mass number predominantly found in nature. Examples of isotopes suitable for inclusion in the compounds of the present disclosure include, but are not limited to, isotopes of hydrogen (e.g., deuterium ($^2H$), tritium ($^3H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulphur (e.g., $^{35}S$). Certain isotopically labelled compounds (e.g., those incorporating a radioisotope) of the present disclosure are useful in drug and/or substrate tissue distribution study (e.g., analysis). Radioisotopes tritium (i.e., $^3H$) and carbon-14 (i.e., $^{14}C$) are particularly preferred for their ease of incorporation and detectability. Substitutions with positron emission isotopes (e.g., $^{11}C$, $^{18}F$, $^{15}O$, and $^{13}N$) may be used to test substrate receptor occupancy in positron emission tomography (PET) studies. The isotopically-labelled compounds of the present disclosure may be prepared by methods analogous to those described in the accompanying routes and/or examples and preparations by replacing a non-isotopically labelled reagent with an appropriate isotopically labelled reagent. Pharmaceutically acceptable solvates of the present disclosure include those in which the crystallisation solvent may be substituted with an isotope, for example, $D_2O$, acetone-$d_6$, or DMSO-$d_6$.

The term "stereoisomer" means an isomer formed due to at least one asymmetric centre. In a compound with one or more (e.g., one, two, three, or four) asymmetric centres, its exo/meso mixtures, single enantiomers, and diastereomer mixtures and individual diastereomers may be produced. Specific individual molecules may also exist as geometric isomers (cis/trans). Similarly, the compound of the present disclosure may exist in a mixture of two or more structurally different forms (commonly referred to as tautomers) in rapid equilibrium. Representative examples of tautomers include keto-enol tautomers, phenol-keto tautomers, nitroso-oxime tautomers, imine-enamine tautomers, and the like. For example, nitroso-oximes may exist in equilibrium in the following tautomeric forms in solution:

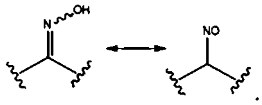

It should be understood that the scope of the present disclosure encompasses all such isomers or mixtures thereof in any proportions (for example, 60%, 65%, 70%, 75%, 80%, 85%, 90%, 95%, 96%, 97%, 98%, 99%).

A solid line (-), a solid wedge (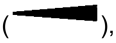), or a dashed wedge (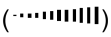) may be used herein to depict chemical bonds of the compounds of the present disclosure. The use of solid lines to depict bonds to asymmetric carbon atoms is intended to indicate that all possible stereoisomers at that carbon atom (e.g., specific enantiomers or racemic mixtures) are included. The use of solid or dashed wedges to depict bonds to asymmetric carbon atoms is intended to indicate that the stereoisomers shown exist. When present in a racemic mixture, solid and dashed wedges are used to define relative stereochemistry, rather than absolute stereochemistry. Unless otherwise specified, the compound of the present disclosure is intended to exist in the form of stereoisomers (including cis and trans isomers, optical isomers (such as R and S enantiomers), diastereomers, geometric isomers, rotamers, conformational isomers, atropisomers, and mixtures thereof). The compound of the present disclosure may exhibit more than one type of isomerism and be composed of mixtures thereof (e.g., racemic mixtures and diastereomeric pairs).

The present disclosure encompasses all possible crystalline forms or polymorphs of the compound of the present disclosure, which may be a single polymorph or a mixture of more than one polymorph at any ratio.

Eutecticum refers to the fact that the active molecules of a drug and additional physiologically acceptable molecules of acids, bases, salts, and non-ionic compounds are connected by hydrogen bonds, $\pi$-$\pi$ stacking, van der Waals forces, and additional non-covalent bonds to be combined in the same crystal lattice.

It should also be understood that some compounds of the present disclosure may exist in free form for treatment, or, where appropriate, in the form of pharmaceutically acceptable derivatives thereof. In the present disclosure, pharmaceutically acceptable derivatives include, but are not limited to, pharmaceutically acceptable salts, esters, solvates, N-oxides, metabolites, or prodrugs, which, after being administered to patients in need thereof, can directly or indirectly provide the compound of the present disclosure or metabolites or residues thereof. Therefore, when the "compound of the present disclosure" is referred to herein, it is also intended to encompass the above various derivative forms of the compound.

Pharmaceutically acceptable salts of the compound of the present disclosure include both acid addition salts and base addition salts, for example, hexafluorophosphate, and meglumine salt. For a review of suitable salts, see Stahl and Wermuth, "Handbook of Pharmaceutical Salts: Properties, Selection, and Use" (Wiley-VCH, 2002).

As used herein, the term "ester" means an ester derived from the compound of each general formula in the present disclosure, which includes physiologically hydrolysable esters (hydrolysable under physiological conditions to release the compound of the present disclosure in the form of free acid or alcohol). The compound of the present disclosure itself may also be an ester itself.

The compound of the present disclosure may exist in the form of solvate (preferably hydrate), where the compound of the present disclosure comprises a polar solvent as a structural element of the crystal lattice of the compound, especially, for example, water, methanol, or ethanol. The amount of the polar solvent, especially water, may be stoichiometric or non-stoichiometric.

Those skilled in the art will understand that, since available lone pairs of electrons are required to oxidize nitrogen to form oxides, not all nitrogen-containing heterocyclic rings can form N-oxides. Those skilled in the art can recognize nitrogen-containing heterocyclic rings that can form N-oxides. Those skilled in the art can also recognize that tertiary amines can form N-oxides. The synthesis methods for the preparation of *N*-oxides of heterocyclic rings and tertiary amines are well known to those skilled in the art, including but not limited to the use of peroxyacids such as peroxyacetic acid and m-chloroperoxybenzoic acid (MCPBA), hydrogen peroxide, alkyl hydroperoxides such as tert-butyl hydroperoxide and sodium perborate, and dioxirane such as dimethyl dioxirane to oxidize heterocyclic rings and tertiary amines. These methods for the preparation of N-oxides have been widely described and summarized in literature, for example, T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The present disclosure further includes, within its scope, metabolites of the compound of the present disclosure, i.e., substances formed in vivo from the administered compound of the present disclosure. Such products may be produced by, for example, oxidation, reduction, hydrolysis, amidation, deamidation, esterification, enzymolysis, and the like of the administered compound. Therefore, the present disclosure includes metabolites of the compound of the present disclosure, including compounds prepared by exposing a mammal to the compound of the present disclosure for a period of time sufficient to produce its metabolites.

The present disclosure further includes, within its scope, the prodrugs of the compound of the present disclosure,

which are certain derivatives of the compound of the present disclosure that may themselves have less pharmacological activity or no pharmacological activity, and that may be converted into the compound of the present disclosure having the desired activity by, for example, hydrolytic cleavage when administered into or onto the human body. Generally, such prodrugs are functional group derivatives of the compound, which are easily converted into the desired therapeutically active compound in vivo. Additional information on the use of prodrugs may be found in "Pro-drugs as Novel Delivery Systems", Volume 14, ACS Symposium Series (T. Higuchi and V. Stella). The prodrugs of the present disclosure may be prepared by, for example, substituting appropriate functional groups present in the compound of the present disclosure with certain moieties known to those skilled in the art as "pro-moiety (for example, as described in "Design of Prodrugs", H. Bundgaard (Elsevier, 1985))".

The present disclosure further includes the compound of the present disclosure comprising protective groups. In any process of preparing the compound of the present disclosure, protection of sensitive groups or reactive groups on any related molecules may be necessary and/or desirable, thereby forming a chemically protected form of the compound of the present disclosure. This may be achieved by conventional protective groups, for example, those protective groups as described in T. W. Greene & P. G. M. Wuts, Protective Groups in Organic Synthesis, John Wiley & Sons, 1991. These references are incorporated herein by reference. The protective groups may be removed at an appropriate subsequent stage using methods known in the art.

The term "about" means within ±10%, preferably within ±5%, more preferably within ±2%, of said value.

"Pharmaceutically acceptable carriers" in the present disclosure refer to diluents, adjuvants, excipients, or vehicles which are administered together with a therapeutic agent, and are, within the scope of sound medical judgement, suitable for contact with the tissues of human beings and/or other animals without excessive toxicity, irritation, allergic reaction, or other problems or complications commensurate with a reasonable benefit/risk ratio.

Pharmaceutically acceptable carriers useful in the pharmaceutical composition of the present disclosure include, but are not limited to, sterile liquid. Examples of suitable pharmaceutically acceptable carriers are as described in Remington's Pharmaceutical Sciences (1990).

The compound of the present disclosure or a pharmaceutical composition comprising it may act systemically and/or locally. For this purpose, they may be administered through a suitable route.

For these administration routes, the compound of the present disclosure or a pharmaceutical composition comprising it may be administered in a suitable dosage form.

The term "effective amount" as used herein refers to an amount of a compound that, after being administered, will relieve one or more symptoms of the disease being treated to a certain extent.

The dosage regimen may be adjusted to provide the optimal desired response. For example, a single bolus may be administered, several divided doses may be administered over time, or the dose may be proportionally reduced or increased as indicated by the exigencies of the treatment. It should be noted that the dose may vary with the type and severity of the condition to be alleviated, and may include single or multiple doses. It should be further understood that for any particular subject, the specific dosage regimen should be adjusted over time according to the needs of the subject and the professional judgement of the person administering the composition or supervising the administration of the composition.

The dosage of the compound of the present disclosure to be administered will depend on the subject being treated, the severity of the disease or condition, the rate of administration, the disposal of the compound, and the judgement of the prescribing physician. In general, the effective dosage ranges from about 0.0001 to about 50 mg per kg body weight per day. In some cases, dosage levels not higher than the lower limit of the aforesaid range may be adequate, while in other cases still larger doses may be employed without causing any harmful side effect, provided that such larger doses are first divided into several smaller doses for administration throughout the day.

The content or amount of the compound of the present disclosure in the pharmaceutical composition may be from about 0.01 mg to about 1,000 mg.

Unless otherwise specified, the term "treating" as used herein means reversing, alleviating, inhibiting the progress of, or preventing the disorder or condition to which such term applies, or one or more symptoms of such disorder or condition.

The "subject" as used herein includes human or non-human animals. Exemplary human subjects include human subjects suffering from diseases (such as the diseases described herein) (referred to as patients) or normal subjects. In the present disclosure, "non-human animals" include all vertebrates, for example, non-mammals (such as birds, amphibians, reptiles) and mammals, for example, non-human primates, livestock, and/or domesticated animals (such as sheep, dogs, cats, cows, and pigs, *etc.).*

In some embodiments, the compound of the present disclosure may be administered in combination with one or more additional therapeutic agents or prophylactic agents (for example, additional drugs for treating a cancer or neoplastic disease). In some embodiments, the method of the present disclosure may also include the administration of one or more additional therapeutic agents or prophylactic agents (e.g., additional drugs for treating a cancer or a neoplastic disease).

**Medical** Use **and Therapeutic Methods**

The present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET-altered tumors:

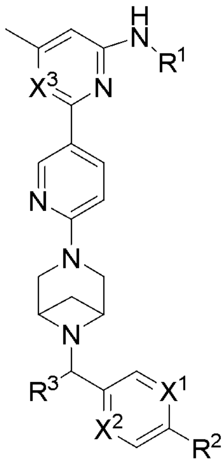

Formula **I**

wherein:

$R^1$ is selected from the group consisting of 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;
$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;
$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and
$X^1$, $X^2$ and $X^3$ are each independently selected from the group consisting of CH and N.

In certain embodiments, the present disclosure provides use of Compound 1, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET-altered tumors, wherein Compound 1 has the following structure:

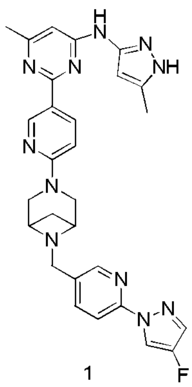

1 ,

for example, the pharmaceutically acceptable salt of Compound 1 is the fumarate salt of Compound 1.

In certain embodiments, the RET alteration is a RET fusion or RET mutation.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET+ solid tumors.

In certain embodiments, the RET+ solid tumor is a RET fusion-positive or RET mutation-positive solid tumor.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E.

In certain embodiments, the RET fusion-positive or RET mutation-positive solid tumor is selected from non-small cell lung cancer (NSCLC), medullary thyroid carcinoma (MTC), papillary thyroid carcinoma (PTC), pancreatic cancer, ovarian cancer, inhibitor resistant solid tumors, and neuroendocrine colorectal cancer (neuroendocrine CRC).

In certain embodiments, the RET fusion-positive or RET mutation-positive solid tumor is selected from non-small cell lung cancer (NSCLC), medullary thyroid carcinoma (MTC), papillary thyroid carcinoma (PTC), pancreatic cancer, ovarian cancer, and inhibitor resistant solid tumors.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of non-small cell lung cancer (NSCLC), medullary thyroid carcinoma (MTC), papillary thyroid carcinoma (PTC), pancreatic cancer, ovarian cancer, inhibitor resistant solid tumors, and/or neuroendocrine colorectal cancer (neuroendocrine CRC).

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of non-small cell lung cancer (NSCLC), medullary thyroid carcinoma (MTC), papillary thyroid carcinoma (PTC), pancreatic cancer, ovarian cancer, inhibitor resistant solid tumors.

In certain embodiments, the medicament is one for treating RET+ non-small cell lung cancer, RET+ medullary thyroid carcinoma, RET+ papillary thyroid carcinoma, RET+ pancreatic cancer, RET+ ovarian cancer, inhibitor resistant solid tumors.

In certain embodiments, the medicament is one for treating RET fusion-positive non-small cell lung cancer, RET fusion-positive medullary thyroid carcinoma, RET fusion-positive papillary thyroid carcinoma, RET fusion-positive pancreatic cancer, RET fusion-positive ovarian cancer, RET fusion-positive neuroendocrine colorectal cancer (neuroendocrine CRC), RET fusion-positive inhibitor resistant solid tumors.

In certain embodiments, the medicament is one for treating RET fusion-positive non-small cell lung cancer, RET fusion-positive medullary thyroid carcinoma, RET fusion-positive papillary thyroid carcinoma, RET fusion-positive pancreatic cancer, RET fusion-positive ovarian cancer, RET fusion-positive inhibitor resistant solid tumors.

In certain embodiments, the RET fusion is selected from KIF5B-RET fusion, CCDC6-RET fusion, NCOA4-RET fusion, RELCH-RET fusion, and ERC1-RET fusion.

In certain embodiments, the medicament is one for treating RET mutation-positive non-small cell lung cancer, RET mutation-positive medullary thyroid carcinoma, RET mutation-positive papillary thyroid carcinoma, RET mutation-positive pancreatic cancer, RET mutation-positive ovarian cancer, RET mutation-positive neuroendocrine colorectal cancer (neuroendocrine CRC), RET mutation-positive inhibitor resistant solid tumors.

In certain embodiments, the medicament is one for treating RET mutation-positive non-small cell lung cancer, RET mutation-positive medullary thyroid carcinoma, RET mutation-positive papillary thyroid carcinoma, RET mutation-positive pancreatic cancer, RET mutation-positive ovarian cancer, RET mutation-positive inhibitor resistant solid tumors.

In certain embodiments, the RET mutation is selected from G810S, G810R, G810C, V804M, V804L, and V804E mutations.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the present disclosure provides use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET+ non-small cell lung cancer (NSCLC) as a first-line or second-line or beyond therapy.

In certain embodiments, the RET+ non-small cell lung cancer (NSCLC) is RET fusion-positive or RET mutation-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In another aspect, the present disclosure provides a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET-altered tumors.

In certain embodiments, the RET-altered tumor is a RET+ solid tumor.

In certain embodiments, the RET+ solid tumor is a RET fusion-positive or RET mutation-positive solid tumor.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In certain embodiments, the RET+ solid tumor is selected from non-small cell lung cancer (NSCLC), medullary thyroid carcinoma (MTC), papillary thyroid carcinoma (PTC), pancreatic cancer, ovarian cancer, neuroendocrine colorectal cancer (neuroendocrine CRC), and inhibitor resistant solid tumors.

In certain embodiments, the RET+ solid tumor is selected from non-small cell lung cancer (NSCLC), medullary thyroid carcinoma (MTC), papillary thyroid carcinoma (PTC), pancreatic cancer, ovarian cancer, and inhibitor resistant solid tumors.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the present disclosure provides a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET+ non-small cell lung cancer (NSCLC) as a first-line or second-line therapy.

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In another aspect, the present disclosure provides a method for treating RET-altered tumors, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof.

In certain embodiments, the daily dose of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is 10-120 mg, such as 10 mg, 20 mg, 40 mg, 60 mg, 90 mg, 120 mg, 20-120 mg, 30-120 mg, 40-120 mg, 50-120 mg, 60-120 mg, 70-120 mg, 80-120 mg, 90-120 mg, 100-120 mg or 110-120 mg.

In certain embodiments, the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is administered at a dosing schedule of once every 1-7 days, for example, once daily, once every 2 days, once every 3 days, once every 4 days, once every 5 days, once every 6 days, or once every 7 days.

In certain embodiments, the dosing schedule is administration once daily at a daily dose of 40-120 mg.

In certain embodiments, the RET-altered tumor is a RET+ solid tumor, such as non-small cell lung cancer (NSCLC), medullary thyroid carcinoma (MTC), papillary thyroid carcinoma (PTC), pancreatic cancer, ovarian cancer, neuroendocrine colorectal cancer (neuroendocrine CRC), inhibitor resistant solid tumor.

In certain embodiments, the RET-altered tumor is a RET+ solid tumor, such as non-small cell lung cancer (NSCLC), medullary thyroid carcinoma (MTC), pancreatic cancer, ovarian cancer, inhibitor resistant solid tumor.

In certain embodiments, the RET-altered tumor is a RET fusion-positive solid tumor, such as non-small cell lung cancer (NSCLC), medullary thyroid carcinoma (MTC), papillary thyroid carcinoma (PTC), pancreatic cancer, ovarian cancer, inhibitor resistant solid tumor, neuroendocrine colorectal cancer (neuroendocrine CRC).

In certain embodiments, the RET fusion-positive solid tumor is RET fusion-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the inhibitor is a RET inhibitor or an MKI inhibitor.

In certain embodiments, the RET inhibitor is selpercatinib, pralsetinib, SY5007, YP01001, or HA121-28.

In certain embodiments, the MKI inhibitor is anlotinib, cabozantinib, apatinib, or CX1003.

In certain embodiments, the objective response rate (ORR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 63%, such as 64%.

In certain embodiments, the objective response rate (ORR) of the subject administered with a therapeutically effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof is at least 70%.

In another aspect, the present disclosure provides a method for treating RET+ non-small cell lung cancer (NSCLC) as a first-line or second-line or beyond therapy, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof.

In certain embodiments, the RET+ non-small cell lung cancer (NSCLC) is RET fusion-positive or RET mutation-positive non-small cell lung cancer (NSCLC).

In certain embodiments, the RET fusion is selected from KIF5B-RET fusion, CCDC6-RET fusion, NCOA4-RET fusion, RELCH-RET fusion, and ERC1-RET fusion.

In certain embodiments, the RET mutation is selected from G810S, G810R, G810C, V804M, V804L, and V804E mutations.

In certain embodiments, the subject is treatment-naive or is systemically pretreated, such as pretreated with anti-PD1/PD-L1 therapy, other RET inhibitor therapy (e.g., selpercatinib, pralsetinib, SY5007, YP01001 or HA121-28), MKI inhibitor therapy (e.g., anlotinib, cabozantinib, apatinib or CX1003), or chemotherapy.

In certain embodiments, the subject is treatment-naive or is systemically pretreated, such as pretreated with anti-PD1/PD-L1 therapy, other RET inhibitor therapy, MKI inhibitor therapy, or chemotherapy.

In certain embodiments, the subject is treatment-naive or is systemically pretreated, such as pretreated with anti-PD1/PD-L1 therapy, other RET selective inhibitor therapy, MKI inhibitor therapy, or chemotherapy.

In certain embodiments, the subject is treatment-naive or is systemically pretreated, such as pretreated with anti-PD1/PD-L1 therapy, selpercatinib, pralsetinib, SY5007, YP01001, HA121-28, anlotinib, cabozantinib, apatinib, CX1003, or chemotherapy.

In certain embodiments, the subject is systemically pretreated, such as pretreated with anti-PD1/PD-L1 therapy, other RET inhibitor therapy (e.g., selpercatinib, pralsetinib, SY5007, YP01001, or HA121-28), MKI inhibitor therapy (e.g., anlotinib, cabozantinib or apatinib, CX1003), or chemotherapy.

In certain embodiments, the subject is systemically pretreated, such as pretreated with anti-PD1/PD-L1 therapy, other RET selective inhibitor therapy (e.g., selpercatinib, pralsetinib, or SY5007), MKI inhibitor therapy (e.g., anlotinib, cabozantinib or apatinib, CX1003), or chemotherapy.

In certain embodiments, the subject is systemically pretreated, such as pretreated with anti-PD1/PD-L1 therapy, other RET selective inhibitor therapy, MKI inhibitor therapy, or chemotherapy.

In certain embodiments, the subject is systemically pretreated, such as pretreated with anti-PD1/PD-L1 therapy, selpercatinib, pralsetinib, SY5007, YP01001, HA121-28, anlotinib, cabozantinib or apatinib, CX1003, or chemotherapy.

In preferred embodiments, the subject has a median prior treatment number selected from 1-15, such as 1-12, such as 1-10, such as 1-9, such as 1-7, such as 1, 2, 3, 4, 5, 6, 7, 8, 9, 10, 11, 12, 13, 14, 15.

In preferred embodiments, the subject has a median prior treatment number selected from 1-9, such as 1, 2, 3, 4, 5, 6, 7, 8, 9.

In certain embodiments, the subject has been previously treated with anti-PD1 or PD-L1 systemic therapy.

In preferred embodiments, the disease control rate (DCR) of the subject is at least 91%.

In preferred embodiments, the disease control rate (DCR) of the subject is at least 97%.

In preferred embodiments, the subject has an objective response rate of at least 63%, and a disease control rate of at least 91%.

In preferred embodiments, the subject has an objective response rate of at least 70%, and a disease control rate of at least 97%.

In certain embodiments, the subject has non-small cell lung cancer (NSCLC) with brain metastasis, bone metastasis, or liver metastasis.

In certain embodiments, the subject has non-small cell lung cancer (NSCLC) with brain metastasis, and further,

the subject has not been previously treated with radiation therapy.

In preferred embodiments, the subject's measurable brain lesions exhibit at least a 47% reduction, such as 80%, 100%.

In preferred embodiments, the subject's measurable brain lesions exhibit at least a 69% reduction.

In preferred embodiments, the subject's measurable brain lesions exhibit at least a 47% reduction, such as 80%, 100%, and the overall central nervous system disease control rate (CNS DCR) is 100%.

In preferred embodiments, the subject's measurable brain lesions exhibit at least a 69% reduction, and the overall central nervous system disease control rate (CNS DCR) is 100%.

In certain embodiments, the subject has been previously treated with other RET inhibitor therapy (e.g., selpercatinib, pralsetinib, SY5007, YP01001, or HA121-28).

In certain embodiments, the subject is a patient who has been previously treated with other RET selective inhibitors and has developed resistance.

In certain embodiments, the resistance is to selpercatinib, pralsetinib, and/or SY5007.

In certain embodiments, the subject who has been previously treated with other RET selective inhibitors has an objective response rate (ORR) of at least 43%, and further, the subject's disease control rate (DCR) is at least 86%.

In certain embodiments, the subject who has been previously treated with other RET selective inhibitors has an objective response rate (ORR) of at least 38%, and further, the subject's disease control rate (DCR) is at least 88%.

In certain embodiments, the subject who has been previously treated with other RET selective inhibitors is a patient positive for KIF5B-RET fusion, CCDC6-RET fusion, NCOA4-RET fusion, RELCH-RET fusion, and/or ERC1-RET fusion.

In certain embodiments, the subject who has been previously treated with other RET selective inhibitors is a patient positive for G810S, G810R, G810C, V804M, V804L, and/or V804E mutations.

In certain embodiments, the subject has been previously treated with MKI inhibitor therapy (e.g., anlotinib, cabozantinib or apatinib, CX1003).

In certain embodiments, the subject has been previously treated with chemotherapy.

In certain embodiments, the subject is not systemically pretreated, such as pretreated with anti-PD1/PD-L1 therapy, other RET selective inhibitor therapy (e.g., selpercatinib or pralsetinib), or chemotherapy.

In some embodiments, the objective response rate (ORR) of the subject who is not systemically pretreated is at least 60%.

In some embodiments, the objective response rate (ORR) of the subject who is not systemically pretreated is at least 74%, such as 76%, 77%.

In some embodiments, the objective response rate (ORR) of the subject who is not systemically pretreated is at least 81%.

In some embodiments, the disease control rate (DCR) of the subject who is not systemically pretreated is at least 92%.

In some embodiments, the disease control rate (DCR) of the subject who is not systemically pretreated is at least 96%.

In certain embodiments, the subject who is not systemically pretreated is selected from a subject who has not been previously treated with other RET selective inhibitors.

In certain embodiments, the subject who has not been previously treated with other RET selective inhibitors is a patient positive for KIF5B-RET fusion, CCDC6-RET fusion, NCOA4-RET fusion, RELCH-RET fusion, and/or ERC1-RET fusion.

In certain embodiments, the subject who has not been previously treated with other RET selective inhibitors is a patient positive for G810S, G810R, G810C, V804M, V804L, and/or V804E mutations.

In another aspect, the present disclosure provides a method for treating a patient with a medicament, comprising administering to a subject an effective amount of the compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, according to one or both of the following dosing regimens:

(1) a daily dose of 10-120 mg, such as 20-120 mg, 30-120 mg, 40-120 mg, 50-120 mg, 60-120 mg, 70-120 mg, 80-120 mg, 90-120 mg, 100-120 mg, or 110-120 mg; and/or

(2) administration once every 1-7 days, such as once daily, once every 2 days, once every 3 days, once every 4 days, once every 5 days, once every 6 days, or once every 7 days;

after treatment, evaluating the subject's indicators to ensure no occurrence of Grade 3 or higher treatment-related adverse events (TRAE), or that majority (e.g., 74.4%) of reversible treatment-related adverse events (TRAE) are less than Grade 3.

In certain embodiments, the "majority" is defined as 76.1%.

In certain embodiments, the treatment-related adverse events less than Grade 3 are selected from elevated aspartate aminotransferase (AST), elevated alanine aminotransferase (ALT), increased creatinine, increased bilirubin, anemia, constipation, and headache.

In certain embodiments, the treatment-related adverse events less than Grade 3 are selected from elevated aspartate aminotransferase (AST), elevated alanine aminotransferase (ALT), increased creatinine, increased bilirubin, constipation, and headache.

In certain embodiments, the treatment-related adverse events of Grade 3 or higher are selected from increased alkaline phosphatase (ALP), increased gamma-glutamyl transferase (GGT), and intestinal obstruction.

In preferred embodiments, hypertension, QT interval prolongation, thrombocytopenia, and lymphocytopenia are rare (e.g., <5%) or of low grade.

In preferred embodiments, hypertension, QT interval prolongation, and lymphocytopenia are rare (e.g., <5%) or of low grade.

In some embodiments, the compound has a structure represented by Formula I-A:

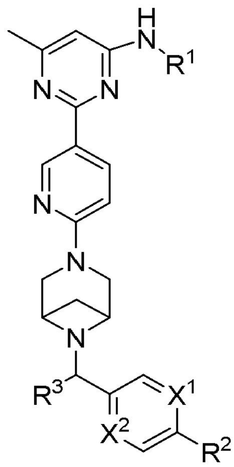

Formula I-A

wherein $R^1$, $R^2$, $R^3$, $X^1$ and $X^2$ are as defined above for Formula I.

In some embodiments, the compound has a structure represented by Formula I-B:

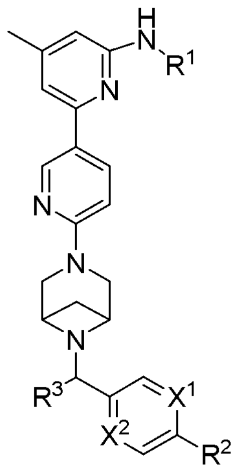

Formula I-B

wherein $R^1$, $R^2$, $R^3$, $X^1$ and $X^2$ are as defined above for Formula I.

In certain embodiments, in the use of the compounds of Formula I, Formula I-A and Formula I-B provided by the present disclosure, $R^1$ is 5-10-membered heteroaryl which is optionally substituted with one or more substituents independently selected from the group consisting of hydroxyl, halogen, CN, $C_{1-4}$ alkyl, $C_{1-4}$ haloalkyl, $C_{3-6}$ cycloalkyl and $C_{3-6}$ cycloalkoxy.

In certain embodiments, in the use of the compounds of Formula I, Formula I-A and Formula I-B provided by the present disclosure, $R^1$ is 5-6-membered heteroaryl which is optionally substituted with one or more substituents

independently selected from the group consisting of halogen, $C_{1-3}$ alkyl, and $C_{3-6}$ cycloalkyl.

In certain embodiments, in the use of the compounds of Formula I, Formula I-A and Formula I-B provided by the present disclosure, $R^1$ is pyrazolyl which is optionally substituted with one or more substituents independently selected from the group consisting of halogen, $C_{1-3}$ alkyl (e.g., methyl) and $C_{3-6}$ cycloalkyl (e.g., cyclopropyl).

In certain embodiments, in the use of the compounds of Formula I, Formula I-A and Formula I-B provided by the present disclosure, $R^1$ is pyrazolyl substituted with methyl (e.g., 5-methyl-1H-pyrazol-3-yl or 1-methyl-1H-pyrazol-4-yl) or pyrazolyl substituted with cyclopropyl (e.g., 5-cyclopropyl-1H-pyrazol-3-yl).

In certain embodiments, in the use of the compounds of Formula I, Formula I-A and Formula I-B provided by the present disclosure, $R^2$ is selected from the group consisting of halogen (e.g., Cl), $C_{1-6}$ alkyl (e.g., methyl) and 5-6-membered heteroaryl, wherein the alkyl and heteroaryl are each optionally substituted with one or more substituents independently selected from the group consisting of halogen, $C_{1-4}$ alkyl, $C_{1-4}$ hydroxyalkyl, $C_{1-4}$ heteroalkyl, $C_{3-6}$ cycloalkyl and $C_{3-6}$ cycloalkoxy.

In certain embodiments, in the use of the compounds of Formula I, Formula I-A and Formula I-B provided by the present disclosure, $R^2$ is 5-6-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy.

In certain embodiments, in the use of the compounds of Formula I, Formula I-A and Formula I-B provided by the present disclosure, $R^2$ is 5-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy.

In certain embodiments, in the use of the compounds of Formula I, Formula I-A and Formula I-B provided by the present disclosure, $R^2$ is pyrrolyl, pyrazolyl, imidazolyl, furanyl or thiazolyl, which is optionally substituted with one or more substituents independently selected from the group consisting of F, Cl, methyl,

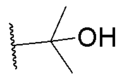

,

cyclopropyl and -O-cyclopropyl.

In certain embodiments, in the use of the compounds of Formula I, Formula I-A and Formula I-B provided by the present disclosure, $R^3$ is selected from H, $C_{1-6}$ alkyl and $C_{3-6}$ cycloalkyl.

In certain embodiments, in the use of the compounds of Formula I, Formula I-A and Formula I-B provided by the present disclosure, $R^3$ is selected from H and $C_{1-6}$ alkyl.

In certain embodiments, in the use of the compounds of Formula I, Formula I-A and Formula I-B provided by the present disclosure, $R^3$ is H or methyl.

In certain embodiments, in the use of the compounds of Formula I, Formula I-A and Formula I-B provided by the present disclosure, $X^1$ is CH or N; and preferably $X^1$ is N.

In certain embodiments, in the use of the compounds of Formula I, Formula I-A and Formula I-B provided by the present disclosure, $X^2$ is CH or N; and preferably, $X^2$ is CH.

In certain embodiments, in the use of the compounds of Formula I provided by the present disclosure, $X^3$ is CH or N; and preferably, $X^3$ is N.

The present disclosure encompasses any combination of the above embodiments.

In some embodiments, the compounds include, but are not limited to:

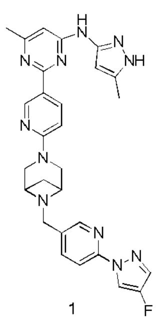

1

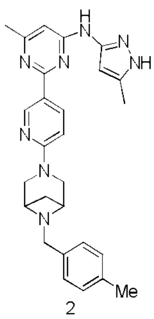

2

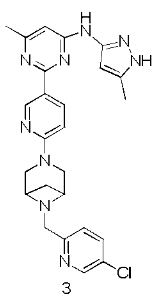

3

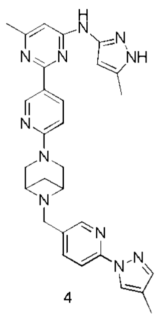

4

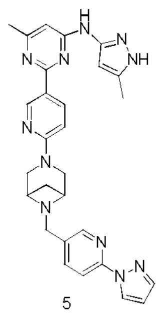

5

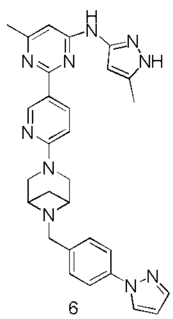

6

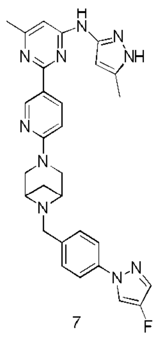

7

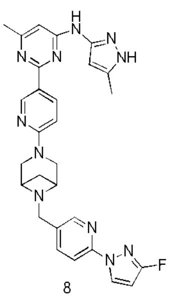
8
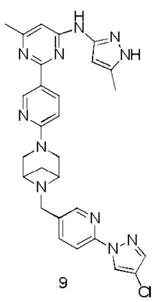
9
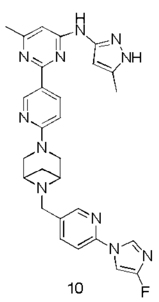
10
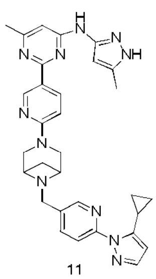
11
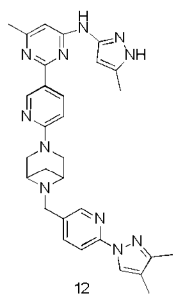
12
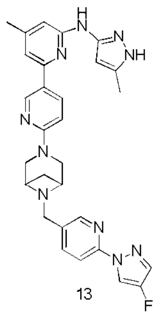
13
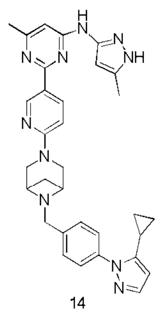
14

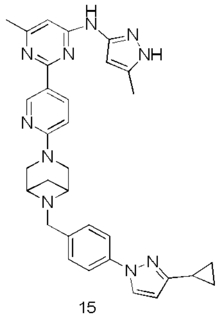
15
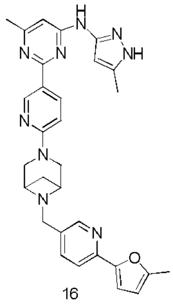
16
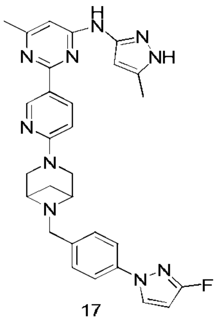
17
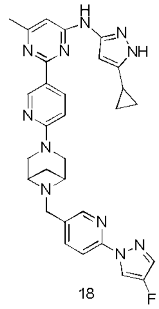
18
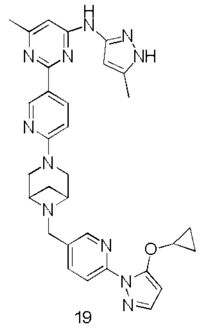
19
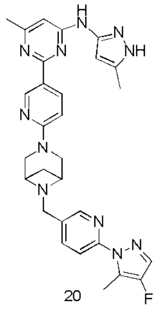
20

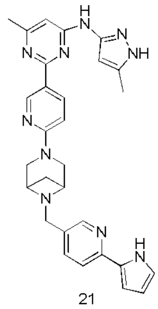
21
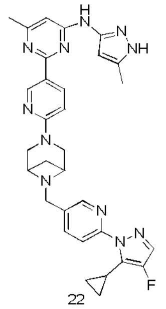
22
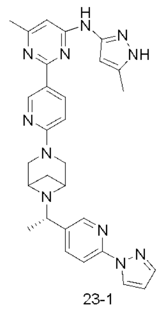
23-1
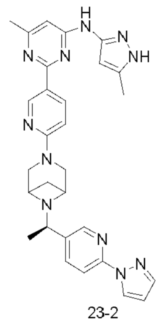
23-2
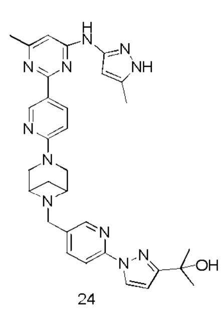
24
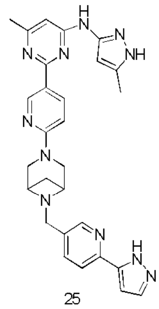
25

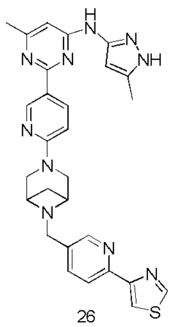
26
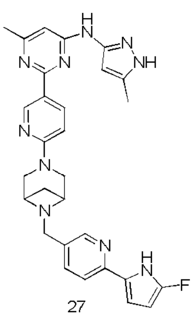
27
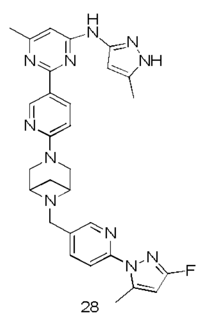
28
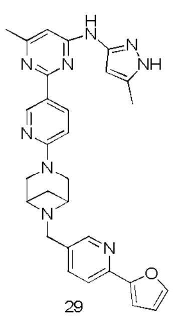
29
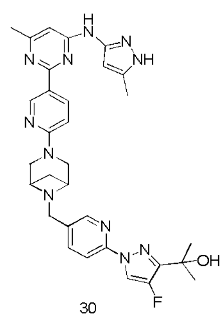
30
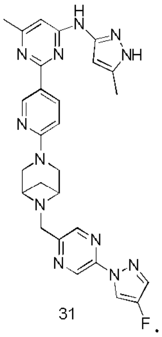
31

In some embodiments, the compound is:

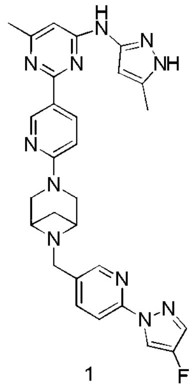

1

In some embodiments, the compound is:

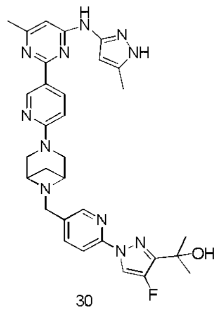

30

In some embodiments, the pharmaceutically acceptable salt is a fumarate.

In some embodiments, the pharmaceutically acceptable salt of the compound is:

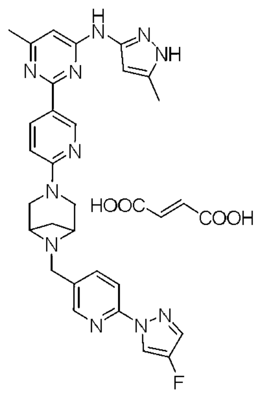

In some embodiments, the pharmaceutically acceptable salt of the compound is:

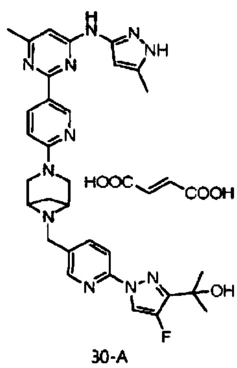

30-A

Examples

The present disclosure will be further described below in combination with examples, but the provision of these examples is not intended to limit the scope of the present disclosure.
The compound of the present disclosure was separated and purified by preparative TLC, silica gel column chromatography, Prep-HPLC, and/or Flash column chromatography, and its structure was validated by $^1$H NMR and/or MS. The reaction was monitored by TLC or LC-MS.
A Bruker superconducting nuclear magnetic resonance spectrometer (model AVACE III HD 400 MHz) was used for $^1$H NMR spectroscopy.
Aglient 1260 Infinity/Aglient 6120 Quadrupole was used for LC/MS.
Silica gel GF 254 was used as the stationary phase for TLC.
200-300 mesh silica gel (Qingdao Marine) was generally used as the stationary phase for column chromatography.
A Biotage flash column chromatograph was used for flash column chromatography.
Agilent 1260, Waters 2489, and GeLai 3500 chromatographs were used for Prep-HPLC.
A BiotageInitiator microwave reactor was used for microwave reaction.
In the following examples, unless otherwise specified, the reaction temperature was room temperature (15-30°C).
Reagents used in the present disclosure were purchased from Acros Organics, Aldrich Chemical Company, or Topbiochem and the like.

Example: Fumarate of 2-(6-(6-((6-(4-fluoro-1H-pyrazol-1-yl)pyridin-3-yl)methyl)-3,6-diazabicyclo[3.1.1]heptan-3-yl) pyridin-3-yl)-6-methyl-N-(5-methyl-1H-pyrazol-3-yl)pyrimidin-4-amine (Compound 1-A)

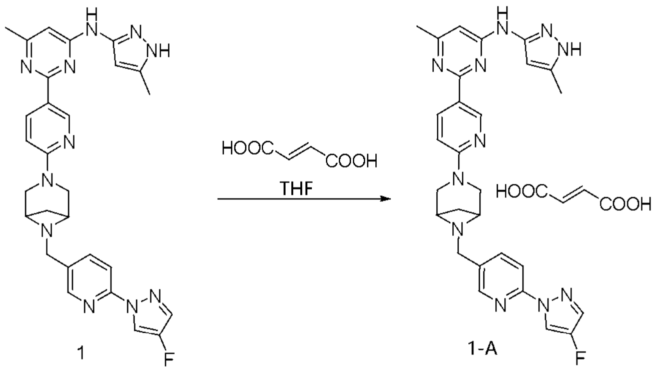


Compound 1 (5 g) was dissolved in 200 mL of THF and stirred until it was completely dissolved. Then 1.56 g of fumaric acid was added slowly, and the reaction mixture was kept for reaction at 20-45°C for 46 h. After completion of the reaction, the mixture was filtered and washed with THF to obtain 7.06 g of a wet solid, which was then dried under vacuum at 40-45°C to give 4.78 g of solid powder.
$^1$H NMR (400 MHz, DMSO-$d_6$) δ 12.43 (br, 3H), 9.66 (s, 1H), 9.13 (*d, J* = 2.4 Hz, 1H), 8.60 *(d, J= 4.4* Hz, 1H), 8.44 (dd, *J* = 8.8, 2.4 Hz, 1H), 8.41 (*d, J* = 1.2 Hz, 1H), 7.97 (dd, *J*= 8.4, 2.0 Hz, 1H), 7.91 *(d, J*= 4.4 Hz, 1H), 7.86 (d, *J* = 8.4 Hz, 1H), 6.81 (br, 1H), 6.76 (d, *J* = 8.8 Hz, 1H), 6.62 (s, 2H), 6.31 (br, 1H), 3.79-3.76 (m, 4H), 3.68-3.58 (m, 4H), 2.62-2.56 (m, 1H), 2.33 (s, 3H), 2.25 (s, 3H), 1.61 (d, *J* = 8.4 Hz, 1H).
The other compounds or salts thereof in the present application can be prepared by the methods as described in WO2022/199503A1 and WO2020/168939A1.

Experimental Evaluation

Experimental Example 1: **RET Inhibition** Experiment

Experimental method: According to the instructions of HTRF KinEASE-TK kit (Cisbio), the inhibitory effect of the compound of the present disclosure on the activity of drug-resistant mutant RET enzymes (RET-G810R, RET-G810S and RET-G810C) was determined. The three drug-resistant mutant RET enzymes were each pre-incubated with different concentrations of test compounds (in the inhibition rate test of the compound: for RET-G810R and RET-G810C, the

compound concentration was 300 and 1,000 nM; for RET-G810S, the compound concentration was 30 and 300 nM. In the $IC_{50}$ test of the compound: for RET-G810R and RET-G810C, the compound concentration was 1-10,000 nM; for RET-G810S, the compound concentration was 0.1-10,000 nM, 9 concentrations in each case). After pre-incubation for 30 min at room temperature, the substrate and adenosine triphosphate (ATP) were added to start the reaction. For RET-G810R and RET-G810S, incubation was carried out at room temperature for 90 min; for RET-G810C, incubation was carried out at room temperature for 120 min. TK antibody-cryptate and streptavidin-XL665 were added, and the test was performed after incubation at room temperature for 60 min. With the solvent group (DMSO) as the negative control and the buffer group (without RET enzyme) as the blank control, the relative inhibitory activity percentage (i.e. inhibition rate) of compounds with different concentrations was calculated as per the following formula:

Relative inhibitory activity percentage = 1 - (compound group of different concentrations - blank control) / (negative control - blank control) * 100%

The relative inhibitory activity percentages of the compounds with different concentrations were plotted with respect to the compound concentrations, and the curve was fitted according to a four-parameter model to calculate the $IC_{50}$ value as per the following formula:

$$y = \min + (\max - \min) / (1 + (x/IC_{50})^{\wedge}(-Hillslope))$$

where y is the relative inhibitory activity percentage, max is the maximum value of the fitted curve, min is the minimum value of the fitted curve, x is the logarithmic concentration of the compound, and Hillslope is the slope of the curve.

The experimental results are shown in Table 1-7.

**Table 1.** Inhibition rates of the compounds of the present disclosure at a concentration of 300 nM on the activity of mutant RET-G810R enzyme

| Compound No. | Inhibition rate on RET-G810R (%) |
|---|---|
| 2 | 70.41±4.57 |
| 3 | 72.49±3.15 |
| 4 | 78.98±0.24 |
| 6 | 77.16±3.87 |
| 9 | 78.59±4.50 |
| 10 | 72.78±1.58 |
| 12 | 69.44+0.31 |

**Table 2.** $IC_{50}$ (nM) for inhibition of RET-G810R enzyme by the compounds of the present disclosure

| Compound No. | $IC_{50}$ (nM) for inhibition of RET-G810R |
|---|---|
| 5 | 185.30 |
| 18 | 132.40 |

As can be seen from Table 1 and Table 2, the compounds of the present disclosure showed significant inhibition on RET-G810R enzyme.

**Table 3.** Inhibition rates of the compounds of the present disclosure at a concentration of 300 nM on the activity of mutant RET-G810S enzyme

| Compound No. | Inhibition rate on RET-G810S (%) |
|---|---|
| 4 | 98.89±0.18 |
| 5 | 97.89±0.57 |
| 6 | 83.01±1.42 |
| 7 | 91.80±0.98 |

(continued)

| Compound No. | Inhibition rate on RET-G810S (%) |
|---|---|
| 8 | 83.73±3.16 |
| 9 | 97.64±0.19 |
| 10 | 88.46±2.23 |
| 11 | 90.87±1.13 |
| 12 | 99.19±0.55 |
| 13 | 97.21±1.01 |
| 14 | 92.56±0.08 |
| 15 | 97.22±0.39 |
| 16 | 85.27±0.37 |
| 17 | 90.64±0.68 |
| 19 | 80.98±7.32 |
| 20 | 96.67±0.03 |
| 25 | 97.26±0.72 |
| 26 | 97.83±0.32 |
| 28 | 90.81±1.24 |
| 29 | 93.21±0.91 |
| 30-A | 99.01±0.15 |
| 31 | 99.69±0.14 |

**Table 4.** $IC_{50}$ (nM) for inhibition of RET-G810S enzyme by the compounds of the present disclosure

| Compound No. | $IC_{50}$ (nM) for inhibition of RET-G810S |
|---|---|
| 18 | 15.94 |
| 21 | 6.61 |
| 24 | 57.21 |
| 27 | 23.11 |

As can be seen from Table 3 and Table 4, the compounds of the present disclosure showed significant inhibition on RET-G810S enzyme.

**Table 5.** Inhibition rates of the compounds of the present disclosure at a concentration of 300 nM on the activity of mutant RET-G810C enzyme

| Compound No. | Inhibition rate on RET-G810C (%) |
|---|---|
| 12 | 69.10±5.56 |
| 19 | 82.80±3.66 |
| 20 | 86.86±1.73 |
| 22 | 84.26±6.82 |
| 27 | 82.66±2.47 |
| 31 | 74.13±1.28 |

**Table 6.** $IC_{50}$ (nM) for inhibition of RET-G810C enzyme by the compounds of the present disclosure

| Compound No. | $IC_{50}$ (nM) for inhibition of RET-G810C |
|---|---|
| 18 | 36.34 |
| 21 | 172.90 |
| 30-A | 124.90 |

As shown in Table 5 and Table 6, the compounds of the present disclosure showed significant inhibition on RET-G810C enzyme.

**Table 7.** $IC_{50}$ (nM) for inhibition of RET mutant enzymes by the compounds of the present disclosure

| Compound No. | $IC_{50}$ (nM) for inhibition of RET-G810R | $IC_{50}$ (nM) for inhibition of RET-G810S | $IC_{50}$ (nM) for inhibition of RET-G810C |
|---|---|---|---|
| BLU-667 | 168.45±0.45 | 1.77±0.27 | 48.81±3.82 |
| LOXO-292 | 73.97±16.40 | 5.66±0.22 | 151.30±30.70 |
| 1-A | 19.62±0.67 | 2.69±0.56 | 53.16±10.67 |

As can be seen from Table 7, Compound 1-A of the present disclosure remarkably inhibited RET-G810R enzyme, RET-G810S enzyme, and RET-G810C enzyme.

**Experimental Example 2: Inhibitory Effect of the Compound on Proliferation** of RET **Mutant cells**

Experimental method: Ba/F3 cells (Ba/F3 KIF5B-$RET^{G810R}$, Ba/F3 KIF5B-$RET^{G810S}$, Ba/F3 KIFSB-$RET^{G810C}$) in logarithmic phase were respectively collected and inoculated into 96-well plates, with 2000 cells/95 μl/well. To the 96-well plate, 5 μl of solutions of BLU-667, Loxo-292 and Compound 1-A were added respectively (final concentration: 1.52-10000 nM). In addition, negative control wells (without the test compound) and blank control wells (without cells) were set. The cells were cultured at 37°C and 5% $CO_2$ for 3 days, then Cell Titer Glo reagent was added at 50 μl/well to lyse the cells, the Luminescence signal value was detected by a microplate reader, and the inhibition rate % was calculated.

Ba/F3 cells (Ba/F3 KIF5B-$RET^{V804E}$, Ba/F3 KIF5B-$RET^{V804L}$, Ba/F3 KIF5B-$RET^{V804M}$) in logarithmic phase were respectively collected and inoculated into 96-well plates, with 1000 cells/95 μl/well. To the 96-well plate, 5 μl of solutions of BLU-667, Loxo-292 and Compound 1-A were added respectively (final concentration: 4~25000 nM). In addition, negative control wells (without the test compound) and blank control wells (without cells) were set. The cells were cultured at 37°C and 5% $CO_2$ for 3 days, then Cell Titer Glo reagent was added at 50 μl/well to lyse the cells, the Luminescence signal value was detected by a microplate reader, and the inhibition rate % was calculated.

Inhibition rate % = [1 - (average signal value of compound group - average signal value of blank control) / (average signal value of negative control - average signal value of blank control)] * 100%; the inhibition rate % was fitted using the four-parameter equation "log(agonist) vs response-variable slope (four parameters)" in GraphPad Prism 6.0, and the $IC_{50}$ was obtained by calculation.

Experimental results: See Table 8. The following results show that Compound 1-A is superior to BLU-667 and Loxo-292 in inhibiting the proliferation of Ba/F3 KIF5B-$RET^{G810R}$, Ba/F3 KIF5B-$RET^{G810S}$, Ba/F3 KIF5B-$RET^{G810C}$, Ba/F3 KIF5B-$RET^{V804E}$, Ba/F3 KIF5B-$RET^{V804L}$, Ba/F3 KIF5B-$RET^{V804M}$ cells.

**Table 8.** Inhibition $IC_{50}$ (nM) of the compounds on the proliferation of the cell

| Cell | BLU-667 | Loxo-292 | 1-A |
|---|---|---|---|
| Ba/F3 KIF5B-$RET^{G810R}$ | 181.50±80.02 | 116.30±1.15 | 26.28±5.76 |
| Ba/F3 KIF5B-$RET^{G810S}$ | 28.96±4.47 | 87.83±0.75 | 20.21±0.35 |
| Ba/F3 KIF5B-$RET^{G810C}$ | 146.40±3.40 | 477.00±4.40 | 65.30±1.00 |
| Ba/F3 KIF5B-$RET^{V804M}$ | 39.47±16.72 | 503.10±189.80 | 25.51±1.91 |
| Ba/F3 KIF5B-$RET^{V804E}$ | 76.18±9.74 | 460.00±32.90 | 59.77±8.33 |
| Ba/F3 KIFSB-$RET^{V804L}$ | 87.11±11.34 | 1334.95±666.05 | 66.62±20.73 |

**Experimental Example 3: Safety Evaluation**

A total of 109 patients with RET-altered tumors were recruited, and compound 1-A of the present disclosure was administered once daily at six dosage levels of 10 mg, 20 mg, 40 mg, 60 mg, 90 mg, and 120 mg. No dose-limiting toxicities (DLT) were observed, nor was the maximum tolerated dose (MTD) reached. The majority (76.1%) of treatment-related adverse events (TRAE) were of grade 1-2 and were reversible, such as elevated aspartate aminotransferase (AST), elevated alanine aminotransferase (ALT), increased creatinine, increased bilirubin, anemia, constipation, and headache.

**Experimental Example 4: Treatment of NSCLC, MTC, Pancreatic Cancer, and Ovarian Cancer**

A total of 90 patients were recruited, including 70 patients with non-small cell lung cancer (NSCLC), 18 patients with medullary thyroid carcinoma (MTC), 1 patient with pancreatic cancer, and 1 patient with ovarian cancer. Compound 1-A of the present disclosure was administered once daily at dosage levels of 40-120 mg (40 mg, 60 mg, 90 mg, and 120 mg), resulting in an objective response rate (ORR) of 60% and a disease control rate (DCR) of 94% for the 90 patients.

Among the 70 NSCLC patients, there were 26 who had not been systemically pretreated, 33 who had previously been systemically pretreated (excluding first-generation selective RET inhibitors), and 11 who had been previously treated with at least first-generation selective RET inhibitor therapy.

Among the 26 NSCLC patients who had not been systemically pretreated, there were 13 patients positive for KIF5B-RET fusion, 7 patients positive for CCDC6-RET fusion, 1 patient positive for NCOA4-RET fusion, and 1 patient positive for RELCH-RET fusion, with an objective response rate (ORR) of 81% (21/26, including 1 complete response (CR) and 20 partial responses (PR), where both the NCOA4-RET fusion-positive and RELCH-RET fusion-positive patients had partial responses (PR)). The disease control rate (DCR) was 96%.

Among the 33 NSCLC patients who had previously been systemically pretreated (excluding first-generation selective RET inhibitors), there were 21 patients positive for KIF5B-RET fusion, 4 patients positive for CCDC6-RET fusion, and 1 patient positive for ERC1-RET fusion.

The median prior treatment number for these 33 patients was 2, ranging from 1 to 9, with 10 patients (30%) previously having been systemically pretreated with anti-PD-1 or PD-L1, and 5 patients (15%) having previously been treated with MKI inhibitor therapy. Among these 33 patients, the objective response rate (ORR) was 70% (23/33), with 1 ERC1-RET fusion-positive patient achieving complete response (CR), and the disease control rate (DCR) was 97%.

11 NSCLC patients were systemically pretreated with first-generation selective RET inhibitors. 8 patients were dosed at either 90 mg or 120 mg, and 3 patients were dosed at either 40 mg or 60 mg. Among the 8 patients dosed at 90 mg or 120 mg, 3 patients achieved partial response (PR), and 4 patients had stable disease (SD), with tumor shrinkage observed in 7 patients (88%).

Among the 70 NSCLC patients, 12 had brain metastasis, with 1 patient having received brain radiation therapy. Six patients had measurable brain lesions, which showed reductions of 100%, 100%, 100%, 69%, 47%, and 17% after treatment, resulting in an overall central nervous system disease control rate (CNS DCR) of 100%.

The above examples do not limit the embodiment of the present disclosure in any way. In addition to those described herein, various modifications of the present disclosure will be apparent to those skilled in the art based on the foregoing description. Such modifications are also intended to fall within the scope of the appended claims. The references cited in the present disclosure (including all patents, patent applications, journal articles, books, and any other publications) are incorporated herein by reference in their entirety.

## Claims

1. Use of a compound of Formula I, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof in the manufacture of a medicament for the treatment of RET altered tumors:

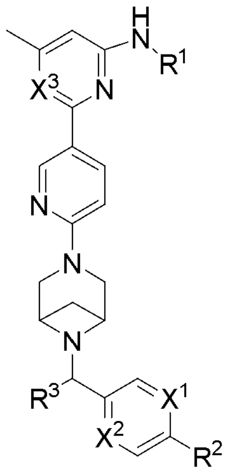

Formula **I**

wherein:

$R^1$ is selected from the group consisting of 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and

$X^1$, $X^2$ and $X^3$ are each independently selected from the group consisting of CH and N.

**2.** The use according to claim 1, wherein the RET-altered tumors are RET+ solid tumors.

**3.** The use according to any one of claims 1-2, wherein the RET-altered tumor is selected from the group consisting of non-small cell lung cancer (NSCLC), medullary thyroid carcinoma (MTC), papillary thyroid carcinoma (PTC), pancreatic cancer, ovarian cancer, inhibitor resistant solid tumors, and neuroendocrine colorectal cancer (neuroendocrine CRC).

**4.** The use according to any one of claims 1-3, wherein the RET-altered tumor is non-small cell lung cancer (NSCLC).

**5.** The use according to any one of claims 1-4, wherein the medicament is for the treatment of RET+ non-small cell lung cancer (NSCLC) as a first-line or second-line or beyond therapy.

**6.** The use according to any one of claims 1-3, wherein the RET-altered tumor is pancreatic cancer.

**7.** The use according to any one of claims 1-3, wherein the RET-altered tumor is ovarian cancer.

**8.** The use according to claim 3, wherein the inhibitor is a RET inhibitor or an MKI inhibitor.

**9.** The use according to claim 8, wherein the RET inhibitor is selpercatinib, pralsetinib, SY5007, YP01001 or HA121-28.

**10.** The use according to claim 8, wherein the MKI inhibitor is anlotinib, cabozantinib, apatinib or

**11.** The use according to claim 9, wherein the RET inhibitor is selpercatinib or pralsetinib.

**12.** The use according to any one of claims 1-11, wherein the RET alteration is a RET mutation or fusion, for example, the RET fusion is selected from KIF5B-RET fusion, CCDC6-RET fusion, NCOA4-RET fusion, RELCH-RET fusion, and

ERC1-RET fusion; and the RET mutation is selected from G810S, G810R, G810C, V804M, V804L, and V804E mutations.

**13.** The use according to any one of claims 1-12, wherein $R^1$ is 5-10-membered heteroaryl which is optionally substituted with one or more substituents independently selected from the group consisting of hydroxyl, halogen, CN, $C_{1-4}$ alkyl, $C_{1-4}$ haloalkyl, $C_{3-6}$ cycloalkyl and $C_{3-6}$ cycloalkoxy;

preferably, $R^1$ is 5-6-membered heteroaryl which is optionally substituted with one or more substituents independently selected from the group consisting of halogen, $C_{1-3}$ alkyl and $C_{3-6}$ cycloalkyl;
preferably, $R^1$ is pyrazolyl which is optionally substituted with one or more substituents independently selected from the group consisting of halogen, $C_{1-3}$ alkyl (e.g., methyl) and $C_{3-6}$ cycloalkyl (e.g., cyclopropyl); and
preferably, $R^1$ is pyrazolyl substituted with methyl (e.g., 5-methyl-1H-pyrazol-3-yl or 1-methyl-1H-pyrazol-4-yl) or pyrazolyl substituted with cyclopropyl (e.g., 5-cyclopropyl-1H-pyrazol-3-yl).

**14.** The use according to any one of claims 1-13, wherein $R^2$ is selected from the group consisting of halogen (e.g., Cl), $C_{1-6}$ alkyl (e.g., methyl) and 5-6-membered heteroaryl, wherein the alkyl and heteroaryl are each optionally substituted with one or more substituents independently selected from the group consisting of halogen, $C_{1-4}$ alkyl, $C_{1-4}$ hydroxyalkyl, $C_{1-4}$ heteroalkyl, $C_{3-6}$ cycloalkyl and $C_{3-6}$ cycloalkoxy;

preferably, $R^2$ is 5-6-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy;
preferably, $R^2$ is 5-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy; and
preferably, $R^2$ is pyrrolyl, pyrazolyl, imidazolyl, furanyl or thiazolyl, which is optionally substituted with one or more substituents independently selected from the group consisting of F, Cl, methyl,

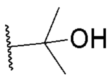,

, cyclopropyl and -O-cyclopropyl.

**15.** The use according to any one of claims 1-14, wherein $R^3$ is selected from the group consisting of H, $C_{1-6}$ alkyl and $C_{3-6}$ cycloalkyl;

preferably, $R^3$ is selected from H and $C_{1-6}$ alkyl; and
preferably, $R^3$ is H or methyl.

**16.** The use according to any one of claims 1-15, wherein:

$X^1$ is CH or N; and preferably, $X^1$ is N;
$X^2$ is CH or N; and preferably, $X^2$ is CH; and/or
$X^3$ is CH or N; and preferably, $X^3$ is N.

**17.** The use according to any one of claims 1-16, wherein the compound has a structure represented by Formula I-A or Formula I-B:

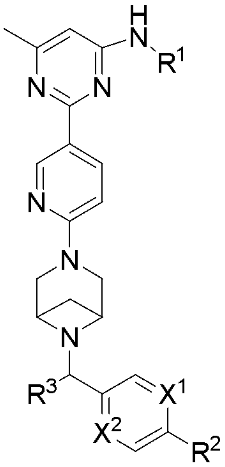

Formula I-A

or

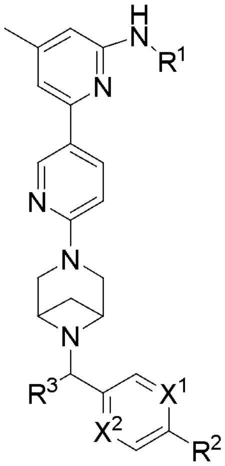

Formula I-B

wherein $R^1$, $R^2$, $R^3$, $X^1$ and $X^2$ are as defined for Formula I.

**18.** The use according to any one of claims 1-17, wherein the compound is selected from the group consisting of:

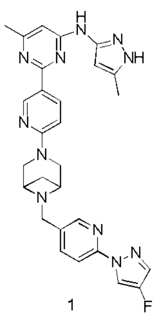

1

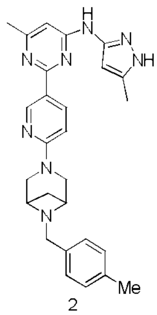

2

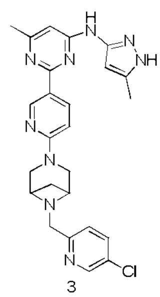

3

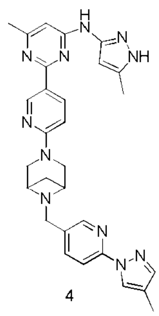

4

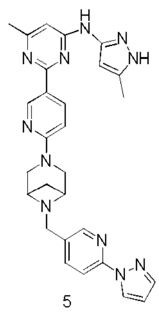

5

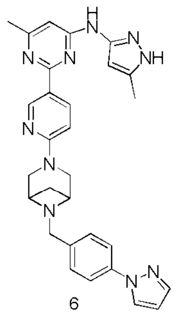

6

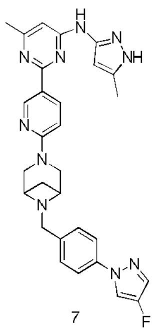

7

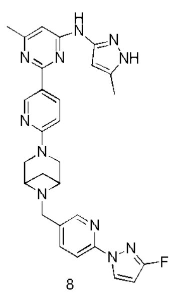

8

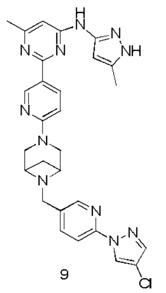

9

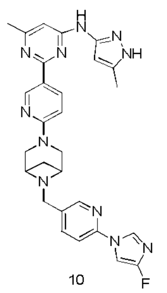

10

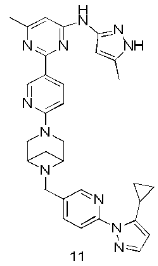

11

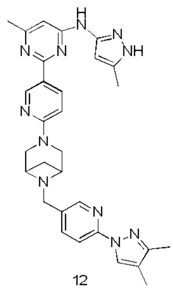

12

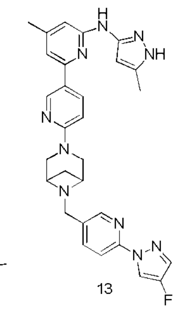

13

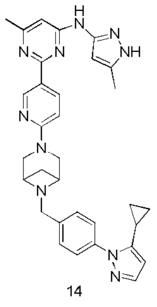

14

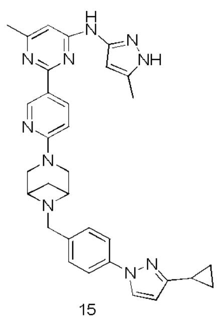

15

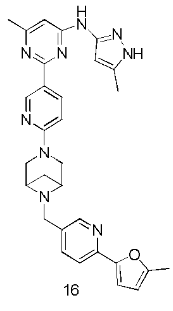

16

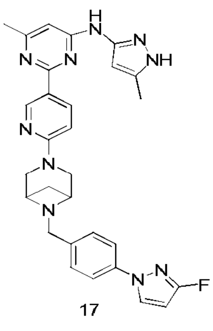

17

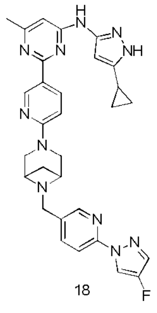

18

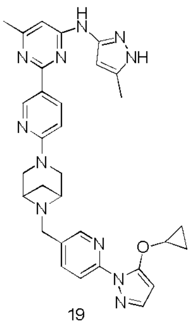

19

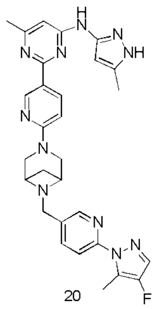
20
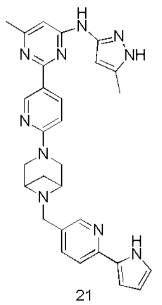
21
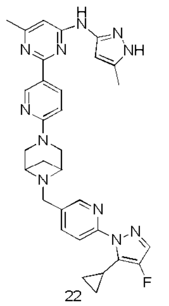
22
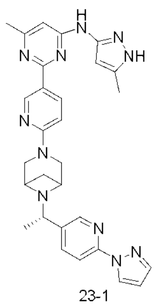
23-1
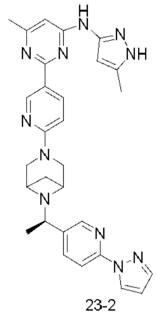
23-2
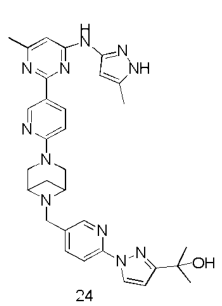
24

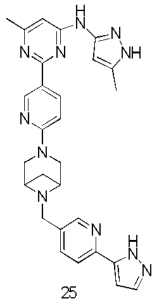
25
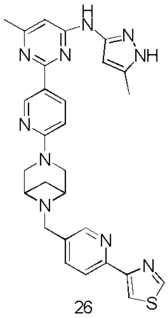
26
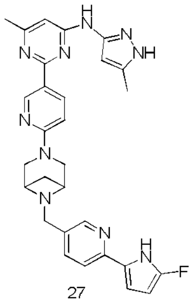
27
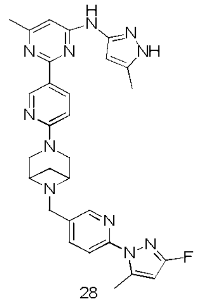
28
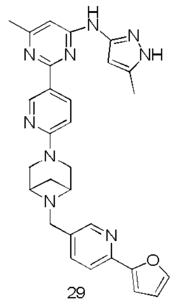
29
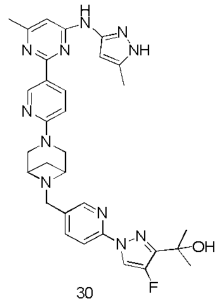
30

and

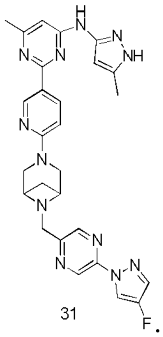
31 .

**19.** The use according to any one of claims 1-18, wherein the pharmaceutically acceptable salt is a fumarate.

**20.** The use according to any one of claims 1-19, wherein the compound is:

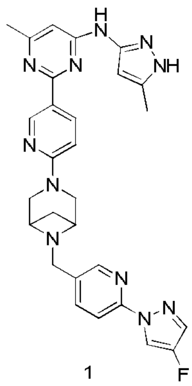
1 or
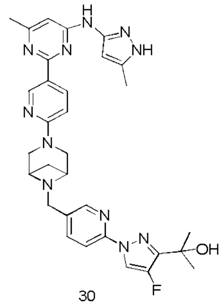
30 .

**21.** The use according to any one of claims 1-20, wherein the pharmaceutically acceptable salt of the compound is:

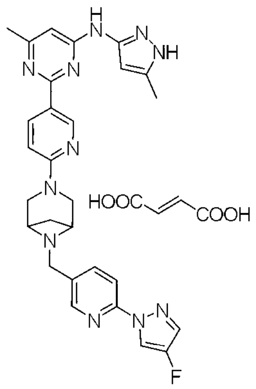

1-A or

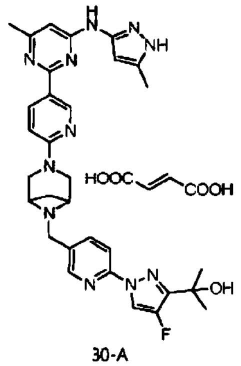

30-A

.

**22.** A method for treating RET-altered tumors, comprising administering to a subject a therapeutically effective amount of the compound as described in the use according to any one of claims 1 and 13-21, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof.

**23.** The method according to claim 22, wherein the RET alteration is a RET mutation or fusion, for example, the RET fusion is selected from KIF5B-RET fusion, CCDC6-RET fusion, NCOA4-RET fusion, RELCH-RET fusion, and ERC1-RET fusion; and the RET mutation is selected from G810S, G810R, G810C, V804M, V804L, and V804E mutations.

**24.** The method according to any one of claims 22-23, wherein the RET-altered tumor is selected from the group consisting of non-small cell lung cancer (NSCLC), medullary thyroid carcinoma (MTC), papillary thyroid carcinoma (PTC), pancreatic cancer, ovarian cancer, inhibitor resistant solid tumors, and neuroendocrine colorectal cancer (neuroendocrine CRC).

**25.** The method according to claim 24, wherein the inhibitor is a RET inhibitor or an MKI inhibitor.

**26.** The method according to claim 25, wherein the RET inhibitor is selpercatinib, pralsetinib, SY5007, YP01001 or HA121-28.

**27.** The method according to claim 25, wherein the MKI inhibitor is anlotinib, cabozantinib, apatinib or CX1003.

**28.** The method according to claim 27, wherein the MKI inhibitor is anlotinib, cabozantinib or apatinib.

**29.** The method according to any one of claims 22-28, wherein the therapeutically effective amount is a daily dose of 10-120 mg, such as 10 mg, 20 mg, 40 mg, 60 mg, 90 mg, 120 mg, 20-120 mg, 30-120 mg, 40-120 mg, 50-120 mg, 60-120 mg, 70-120 mg, 80-120 mg, 90-120 mg, 100-120 mg or 110-120 mg.

**30.** The method according to claim 29, wherein the dosing schedule is once every 1-7 days.

**31.** The method according to any one of claims 22-30, wherein the objective response rate (ORR) of the subject is at least 60%.

**32.** The method according to any one of claims 22-31, wherein the subject is treatment-naive or is systemically pretreated, such as pretreated with anti-PD1/PD-L1 therapy, other RET inhibitor therapy (e.g., selpercatinib, pralsetinib, SY5007, YP01001 or HA121-28), MKI inhibitor therapy (e.g., anlotinib, cabozantinib, apatinib or CX1003), or chemotherapy.

**33.** The method according to any one of claims 22-32, wherein the subject has a median prior treatment number selected from 1-15, such as 1, 2, 3, 4, 5, 6, 7, 8, or 9.

**34.** The method according to any one of claims 22-33, wherein the subject has non-small cell lung cancer (NSCLC) with brain metastasis, bone metastasis or liver metastasis.

**35.** The method according to any one of claims 22-34, wherein the subject has previously received systemic anti-PD1 or PD-L1 therapy.

**36.** The compound as described in the use according to any one of claims 1 and 13-21, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET-altered tumors.

**37.** The compound as described in the use according to any one of claims 1 and 13-21, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET-altered non-small cell lung cancer (NSCLC), medullary thyroid carcinoma (MTC), papillary thyroid carcinoma (PTC), pancreatic cancer, ovarian cancer, inhibitor resistant solid tumors, and/or neuroendocrine colorectal cancer (neuroendocrine CRC); optionally, the inhibitor is a RET inhibitor or MKI inhibitor; further, the RET inhibitor is selpercatinib, pralsetinib, SY5007, YP01001 or HA121-28; and the MKI inhibitor is anlotinib, cabozantinib, apatinib or CX1003.

**38.** The compound as described in the use according to any one of claims 1 and 13-21, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET fusion-positive (e.g., KIF5B-RET fusion) or mutation-positive non-small cell lung cancer (NSCLC), medullary thyroid carcinoma (MTC), papillary thyroid carcinoma (PTC), pancreatic cancer, ovarian cancer, inhibitor resistant solid tumors, and/or neuroendocrine colorectal cancer (neuroendocrine CRC); optionally, the inhibitor is a RET inhibitor or MKI inhibitor; further, the RET inhibitor is selpercatinib, pralsetinib, SY5007, YP01001 or HA121-28; and the MKI inhibitor is anlotinib, cabozantinib, apatinib or CX1003.

**39.** The compound as described in the use according to any one of claims 1 and 13-21, a stereoisomer, tautomer, or a mixture of the stereoisomer and tautomer thereof, an N-oxide thereof, a pharmaceutically acceptable salt, eutecticum, polymorph, or solvate thereof, or a stable isotope derivative, metabolite, or prodrug thereof, for use in treating RET+ non-small cell lung cancer (NSCLC) as a first-line or second-line or beyond therapy, such as for use in treating RET fusion-positive (e.g., KIF5B-RET fusion, CCDC6-RET fusion, NCOA4-RET fusion, RELCH-RET fusion, and ERC1-RET fusion) or mutation-positive (e.g., G810S, G810R, G810C, V804M, V804L, and V804E mutations) non-small cell lung cancer (NSCLC) as a first-line or second-line or beyond therapy.

## INTERNATIONAL SEARCH REPORT

International application No.
**PCT/CN2024/093044**

**A. CLASSIFICATION OF SUBJECT MATTER**

A61K31/506(2006.01)i; C07D487/08(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, NCBI, 四川科伦博泰生物医药股份有限公司, 卿燕, RET, N-氧化物, G810S, G810R, G810C, V804M, V804L, V804E, KIF5B-RET, CCDC6-RET, NCOA4-RET, RELCH-RET, ERC1-RET

**C. DOCUMENTS CONSIDERED TO BE RELEVANT**

| Category* | Citation of document, with indication, where appropriate, of the relevant passages | Relevant to claim No. |
|---|---|---|
| X | WO 2022199503 A1 (SICHUAN KELUN-BIOTECH BIOPHARMACEUTICAL CO., LTD.) 29 September 2022 (2022-09-29)<br>claims 1-17 | 1-39 |
| X | WO 2020168939 A1 (SICHUAN KELUN-BIOTECH BIOPHARMACEUTICAL CO., LTD.) 27 August 2020 (2020-08-27)<br>abstract, and claims 1-12 | 1-39 |
| X | WO 2022022398 A1 (SICHUAN KELUN-BIOTECH BIOPHARMACEUTICAL CO., LTD.) 03 February 2022 (2022-02-03)<br>claims 1-18 | 1-39 |
| A | CN 113853375 A (VORONOI INC. et al.) 28 December 2021 (2021-12-28)<br>entire document | 1-39 |
| A | CN 112574201 A (SICHUAN KELUN-BIOTECH BIOPHARMACEUTICAL CO., LTD.) 30 March 2021 (2021-03-30)<br>entire document | 1-39 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 July 2024** | **02 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/093044**

**C. DOCUMENTS CONSIDERED TO BE RELEVANT**

| Category* | Citation of document, with indication, where appropriate, of the relevant passages | Relevant to claim No. |
|---|---|---|
| A | WO 2021023209 A1 (APPLIED PHARMACEUTICAL SCIENCE, INC.) 11 February 2021 (2021-02-11)<br>entire document | 1-39 |
| A | WO 2020064009 A1 (APPLIED PHARMACEUTICAL SCIENCE, INC.) 02 April 2020 (2020-04-02)<br>entire document | 1-39 |

Form PCT/ISA/210 (second sheet) (July 2022)

## INTERNATIONAL SEARCH REPORT

International application No.
**PCT/CN2024/093044**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-35**
because they relate to subject matter not required to be searched by this Authority, namely:

Claims 22-35 relate to a method for treating a disease as defined in PCT Rule 39.1(iv). However, the following search opinion is still provided on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/093044**

| Patent document cited in search report | Publication date (day/month/year) | Patent family member(s) | Publication date (day/month/year) |
|---|---|---|---|
| WO 2022199503 A1 | 29 September 2022 | EP 4316490 A1 | 07 February 2024 |
| | | JP 2024511389 A | 13 March 2024 |
| | | KR 20230159367 A | 21 November 2023 |
| | | AU 2022242476 A1 | 26 October 2023 |
| WO 2020168939 A1 | 27 August 2020 | KR 20210131314 A | 02 November 2021 |
| | | BR 112021016299 A2 | 13 October 2021 |
| | | SG 11202107848 TA | 30 August 2021 |
| | | MX 2021009971 A | 13 October 2021 |
| | | US 2022144847 A1 | 12 May 2022 |
| | | AU 2020226422 A1 | 09 September 2021 |
| | | CL 2021002205 A1 | 22 April 2022 |
| | | EP 3929198 A1 | 29 December 2021 |
| | | EP 3929198 A4 | 16 November 2022 |
| | | CO 2021011023 A2 | 09 September 2021 |
| | | CA 3130245 A1 | 27 August 2020 |
| | | IL 285378 A | 30 September 2021 |
| | | JP 2022521859 A | 12 April 2022 |
| WO 2022022398 A1 | 03 February 2022 | BR 112023000065 A2 | 31 January 2023 |
| | | US 2023295174 A1 | 21 September 2023 |
| | | KR 20230044358 A | 04 April 2023 |
| | | JP 2023535361 A | 17 August 2023 |
| | | EP 4190781 A1 | 07 June 2023 |
| | | EP 4190781 A4 | 27 December 2023 |
| CN 113853375 A | 28 December 2021 | KR 20200134179 A | 01 December 2020 |
| | | AU 2020279686 A1 | 16 December 2021 |
| | | IL 288242 A | 01 January 2022 |
| | | SG 11202112796 YA | 30 December 2021 |
| | | EP 3974432 A1 | 30 March 2022 |
| | | EP 3974432 A4 | 28 June 2023 |
| | | WO 2020235945 A1 | 26 November 2020 |
| | | CA 3140067 A1 | 26 November 2020 |
| | | MX 2021014273 A | 06 January 2022 |
| | | BR 112021023282 A2 | 04 January 2022 |
| | | EA 202193049 A1 | 14 February 2022 |
| | | JP 2022533710 A | 25 July 2022 |
| | | JP 7477846 B2 | 02 May 2024 |
| | | US 2022324874 A1 | 13 October 2022 |
| CN 112574201 A | 30 March 2021 | None | |
| WO 2021023209 A1 | 11 February 2021 | KR 20220042293 A | 05 April 2022 |
| | | JP 2022543713 A | 14 October 2022 |
| | | US 2022332731 A1 | 20 October 2022 |
| | | EP 3992197 A1 | 04 May 2022 |
| | | EP 3992197 A4 | 30 August 2023 |
| WO 2020064009 A1 | 02 April 2020 | KR 20210070286 A | 14 June 2021 |
| | | JP 2022508533 A | 19 January 2022 |
| | | JP 7493251 B2 | 31 May 2024 |
| | | EP 3845531 A1 | 07 July 2021 |
| | | EP 3845531 A4 | 24 November 2021 |
| | | US 2021379056 A1 | 09 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310567443


### Non-patent literature cited in the description


- Handbook of Pharmaceutical Salts: Properties, Selection, and Use. **STAHL** ; **WERMUTH**. Wiley-VCH. 2002
- **T. L. GILCHRIST**. Comprehensive Organic Synthesis. Academic Press, vol. 7, 748-750
- **G. W. H. CHEESEMAN** ; **E. S. G. WERSTIUK**. Advances in Heterocyclic Chemistry. Academic Press, vol. 22, 390-392
- **T. HIGUCHI** ; **V. STELLA**. Pro-drugs as Novel Delivery Systems. *ACS Symposium Series*, vol. 14
- **H. BUNDGAARD**. Design of Prodrugs. Elsevier, 1985
- **T. W. GREENE** ; **P. G. M. WUTS**. Protective Groups in Organic Synthesis. John Wiley & Sons, 1991
- Remington's Pharmaceutical Sciences. 1990